# EUROPEAN PATENT APPLICATION

(11) **EP 2 428 216 A2**
(43) Date of publication of application: **14.03.2012**
(21) Application number: 11184922.0
(22) Date of filing: 20.05.2005
(51) Int. Cl.: A61K 38/20, A61K 39/395, C07K 16/28, C07K 16/32

(54) **Methods of treating cancer using IL-21 and monoclonal antibody therapy**

(30) Priority: 20.05.2004 US 572973 P; 10.12.2004 US 635380 P; 14.04.2005 US 671281 P; 12.05.2005 US 680447 P
(62) Divisional of application: 05756320.7
(71) Applicant: ZymoGenetics, Inc., Seattle, WA 98102 (US)
(72) Inventor: Hughes, Steven D., Kenmore, WA 98028 (US); Kindsvogel, Wayne R., Seattle, WA 98115 (US); Holly, Richard D., Seattle, WA 98177 (US); Clegg, Christopher H., Seattle, WA 98177 (US); Foster, Donald C., Lake Forest Park, WA 98155 (US); Johnson, Rebecca A., Seattle, WA 98136 (US); Heipel, Mark D., Seattle, WA 98117 (US); Sivakumar, Pallavur V., Seattle, WA 98112 (US)
(74) Representative: Griffin, Philippa Jane

(57) **Abstract**

There is provided a monoclonal antibody that binds to binds to cytotoxic T lymphocyte-associated antigen 4 (CTLA-4), programmed death-1 (PD-1), B and T lymphocyte attenuator (BTLA), IL-10, TGFβ, or CD25, and an IL-21 polypeptide or fragment of an IL-21 polypeptide as shown in SEQ ID NO:2 from amino acid residue 30 to residue 162, for use in treating cancer in a subject.

## Description

### BACKGROUND OF THE INVENTION

Cytokines generally stimulate proliferation or differentiation of cells of the hematopoietic lineage or participate in the immune and inflammatory response mechanisms of the body. The interleukins are a family of cytokines that mediate immunological responses. Central to an immune response is the T cell, which produces many cytokines and effects adaptive immunity to antigens. Cytokines produced by the T cell have been classified as TH1 and TH2 (Kelso, A. Immun. Cell Biol. 76:300-317, 1998). Type 1 cytokines include IL-2, IFN-γ, LT-α, and are involved in inflammatory responses, viral immunity, intracellular parasite immunity and allograft rejection. Type 2 cytokines include IL-4, IL-5, IL-6, IL-10 and IL-13, and are involved in humoral responses, helminth immunity and allergic response. Shared cytokines between Type 1 and 2 include IL-3, GM-CSF and TNF-α. There is some evidence to suggest that Type 1 and Type 2 producing T cell populations preferentially migrate into different types of inflamed tissue.

Natural killer (NK) cells have a common progenitor cell with T cells and B cells, and play a role in immune surveillance. NK cells, which comprise up to 15% of blood lymphocytes, do not express antigen receptors, and are a component of innate immunity. NK cells are involved in the recognition and killing of tumor cells and virally infected cells. *In vivo,* NK cells are believed to require activation, however, *in vitro,* NK cells have been shown to kill some types of tumor cells without activation.

IL-21 has been shown to be a potent modulator of cytotoxic T cells and NK cells. (Parrish-Novak, et al. Nature 408:57-63, 2000; Parrish-Novak, et al., J. Leuk. Bio. 72:856-863, 2002; Collins et al., Immunol. Res. 28:131-140, 2003; Brady, et al. J. Immunol.:2048-58, 2004.) IL-21 has been shown to co-stimulate the expansion of NK cells, and it has been demonstrated to enhance the effector functions of these cells. T cell responses include enhancement of primary antigen response as modulation of memory T cell functions (Kasaian et al., Immunity 16:559-569, 2002.)

Antibody therapy utilizes antigens that are selectively expressed on certain cell types. Antibody therapy has been particularly successful in cancer treatment because certain tumors either display unique antigens, lineage-specific antigens, or antigens present in excess amounts relative to normal cells. The development of monoclonal antibody (MAb) therapy has evolved from mouse hybridoma technology (Kohler et al., Nature 256:495-497, 1975), which had limited therapeutic utility due to an inability to stimulate human immune effector cell activity and production of human antimouse antibodies (HAMA; Khazaeli et al., J. Immunother. 15:42-52, 1994). Engineering chimeric antibodies which were less antigenic was achieved using human constant regions and mouse variable regions. These antibodies had increased effector functions and reduced HAMA responses (Boulianne et al., Nature 312:643-646, 1984). Human monoclonal antibodies have developed using phage display technology (McCafferty et al., Nature 348:552-554, 1990), and more recently, transgenic mice carrying human Ig loci have been used to produce fully human monoclonal antibodies (Green, J. Immunol. Methods 231:11-23, 1999). For a review of monoclonal antibody therapy, see, Brekke et al., Nat. Rev. Drug Discov. 2:52-62, 2002.

The present invention provides methods for enhancing the antitumor activity of monoclonal antibody therapy with IL-21. The combination of IL-21 and therapeutic monoclonal antibodies provide improvements over monoclonal antibody therapy alone, in particular for patients that do not respond to monoclonal antibody therapy alone or in combination with other treatment regimes. These and other uses should be apparent to those skilled in the art from the teachings herein.

### SUMMARY OF THE INVENTION

The present invention provides a method of treating cancer in a subject, particularly human subjects, comprising co-administering a therapeutically effective amount of a monoclonal antibody and a therapeutically effective amount of an IL-21 polypeptide or fragment of an IL-21 polypeptide as shown in SEQ ID NO:2 from amino acid residue 30 to residue 162. In one embodiment, the monoclonal antibody is an anti-CD20 monoclonal antibody. In another embodiment, the monoclonal antibody is rituximab. In another embodiment, methods of the present invention treat non-Hodgkin's lymphoma. Further embodiments of the present invention provide methods where monoclonal antibody rituximab and IL-21 polypeptide are administered once weekly for up to eight consecutive weeks. In another embodiment, the rituximab is administered once weekly and the IL-21 polypeptide is administered up to five times weekly for up to eight consecutive weeks. Another embodiment of present invention provides that the IL-21 polypeptide dose is from 10 to 500 µg/kg/dose. In certain embodiments of the present invention, the patient has previously been treated with rituximab and showed no appreciable tumor remission or regression. In other embodiments, the patient has relapsed after receiving rituximab therapy.

In another aspect, the present invention provides a method of treating cancer in a subject comprising co-administering a therapeutically effective amount of an anti-CD20 monoclonal antibody and a therapeutically effective amount of an IL-21 polypeptide or a fragment of an IL-21 polypeptide as shown in SEQ ID NO:2 from amino acid residue 30 to residue 162, wherein administering the IL-21 results in an optimal immunological response.

In another aspect, the present invention provides a method treating cancer in a subject comprising co-administering a monoclonal antibody that binds to a Her-2/neu receptor and an IL-21 polypeptide or a fragment of an IL-21 polypeptide as shown in SEQ ID NO:2 from amino acid residue 30 to residue 162. In one embodiment, the subject is a human patient. In another embodiment, the monoclonal antibody is trastuzumab.

One aspect of the present invention provides a method of treating cancer in a subject comprising co-administering a monoclonal antibody that binds to a cytotoxic T lymphocyte-associated antigen 4 (CTLA-4) and an IL-21 polypeptide or a fragment of an IL-21 polypeptide as shown in SEQ ID NO:2 from amino acid residue 30 to residue 162. In certain embodiments, the subject is a human patient. In another embodiment of the present invention, the anti-CTLA-4 monoclonal antibody is administered at a dose of 3 mg/kg every three weeks for four cycles and the IL-21 polypeptide or fragment is administered one to five times weekly for up to eight weeks. The present invention also provides embodiments where the IL-21 polypeptide dose is from 10 to 500 µg/kg/dose.

### BRIEF DESCRIPTION OF THE FIGURES

Figure 1-illustrates survival curves for macrophage-depleted mice were significantly different from non-depleted mice.
Figure 2-illustrates that mice with granulocytes depleted by anti-Gr-1 MAb injections show reduced survival when compared to non-depleted mice.

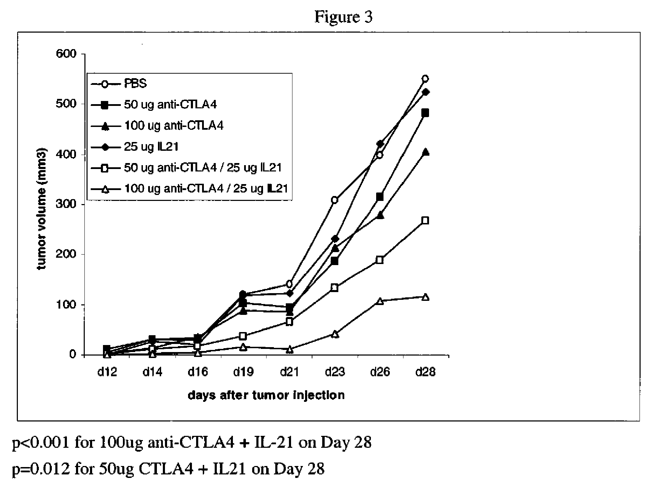

Figure 3-illustrates the combination of anti-CTLA4 + IL21 has antitumor effects in RENCa model.

### DESCRIPTION OF THE INVENTION

Prior to setting forth the invention in detail, it may be helpful to the understanding thereof to define the following terms:
The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a polyhistidine tract, protein A (Nilsson et al., EMBO J. 4:1075, 1985; Nilsson et al., Methods Enzymol. 198:3, 1991), glutathione S transferase (Smith and Johnson, Gene 67:31, 1988), Glu-Glu affinity tag (Grussenmeyer et al., Proc. Natl. Acad. Sci. USA 82:7952-4, 1985), substance P, Flag™ peptide (Hopp et al., Biotechnology 6:1204-10, 1988), streptavidin binding peptide, or other antigenic epitope or binding domain. See, in general, Ford et al., Protein Expression and Purification 2: 95-107, 1991. DNAs encoding affinity tags are available from commercial suppliers (e.g., Pharmacia Biotech, Piscataway, NJ).

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "cancer" or "cancer cell" is used herein to denote a tissue or cell found in a neoplasm which possesses characteristics which differentiate it from normal tissue or tissue cells. Among such characteristics include but are not limited to: degree of anaplasia, irregularity in shape, indistinctness of cell outline, nuclear size, changes in structure of nucleus or cytoplasm, other phenotypic changes, presence of cellular proteins indicative of a cancerous or pre-cancerous state, increased number of mitoses, and ability to metastasize. Words pertaining to "cancer" include carcinoma, sarcoma, tumor, epithelioma, leukemia, lymphoma, polyp, and scirrus, transformation, neoplasm, and the like.

The term "co-administration" is used herein to denote that an IL-21 polypeptide or protein and a therapeutic monoclonal antibody may be given concurrently or at different times. The co-adminstration may be a single co-administration of both IL-21 and monoclonal antibody or multiple cycles of co-administration. Co-administration need not be the only times either IL-21 or the monoclonal antibody is administered to a patient and either agent may be administered alone or in a combination with therapeutic agents other than IL-21.

The term "combination therapy" is used herein to denote that a subject is administered at least one therapeutically effective dose of an IL-21 composition ("IL-21") and a therapeutic monoclonal antibody. The IL-21 composition may be a mature polypeptide, fragment thereof, fusion or conjugate that demonstrates IL-21 biological activity.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, Nature 316:774-78, 1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. When used in this context, the term "isolated" does not exclude the presence of the same polypeptide in alternative physical forms, such as dimers or alternatively glycosylated or derivatized forms.

The term "level" when referring to immune cells, such as NK cells, T cells, in particular cytotoxic T cells, B cells and the like, an increased level is either increased number of cells or enhanced activity of cell function.

The term "level" when referring to viral infections refers to a change in the level of viral infection and includes, but is not limited to, a change in the level of CTLs or NK cells (as described above), a decrease in viral load, an increase antiviral antibody titer, decrease in serological levels of alanine aminotransferase, or improvement as determined by histological examination of a target tissue or organ. Determination of whether these changes in level are significant differences or changes is well within the skill of one in the art.

The term "neoplastic", when referring to cells, indicates cells undergoing new and abnormal proliferation, particularly in a tissue where in the proliferation is uncontrolled and progressive, resulting in a neoplasm. The neoplastic cells can be either malignant, i.e. invasive and metastatic, or benign.

The term "optimal immunological dose" is defined as the dose of IL-21 or IL-21 in combination with a monoclonal antibody that achieves the optimal immunological response.

The term "optimal immunological response" refers to a change in an immunological response after administration of IL-21 or the IL-21 + MAb combination over that seen when the MAb alone is administered, and can be (1) an increase in the numbers of activated or tumor specific CD8 T cells, (2) an increase in the numbers of activated or tumor specific CD8 T cells expressing higher levels of granzyme B or perforin or IFNγ, (3) upregulation of Fcγ receptor (CD 16, CD32, or CD64) on Nk cells, monocytes, or neutrophils, (4) an increase in soluble CD25 in the serum, (5) reduction in serum level of proteins liberated by tumor cells, (see, Taro et al., J. Cell Physiol 203(1):1-5, 2005), for example, carcinoembryonic antigen (CEA), IgG, CA-19-9, or ovarian cancer antigen (CA125), (6) an increase in the numbers of NK cells expressing higher levels of granzyme B, perforin or IFNγ, (7) increase in the levels of activation cytokines such as IL-18, IL-15, IFNγ and chemokines that enable homing of effector cells to the tumor, such as IP-10, RANTES, IL-8, MIP1a or MIP1b, (8) an increase in the numbers of activated macrophages in the periphery or at the tumor site, where activation can be detected by expression of increased MHC class I or Class II, production of DL-15, IL-18, IFNγ, or IL-21, or (9) macrophage activity as indicated by decline in red blood cell count (severity of anemia).

A "polynucleotide" is a single- or double-stranded polymer of deoxyribonucleotide or ribonucleotide bases read from the 5' to the 3' end. Polynucleotides include RNA and DNA, and may be isolated from natural sources, synthesized *in vitro,* or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired.

A "polypeptide" is a polymer of amino acid residues joined by peptide bonds, whether produced naturally or synthetically. Polypeptides of less than about 10 amino acid residues are commonly referred to as "peptides".

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The term "receptor" denotes a cell-associated protein that binds to a bioactive molecule (i.e., a ligand) and mediates the effect of the ligand on the cell. Membrane-bound receptors are characterized by a multi-peptide structure comprising an extracellular ligand-binding domain and an intracellular effector domain that is typically involved in signal transduction. Binding of ligand to receptor results in a conformational change in the receptor that causes an interaction between the effector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids. In general, receptors can be membrane bound, cytosolic or nuclear; monomeric (e.g., thyroid stimulating hormone receptor, beta-adrenergic receptor) or multimeric (e.g., PDGF receptor, growth hormone receptor, IL-3 receptor, GM-CSF receptor, G-CSF receptor, erythropoietin receptor and IL-6 receptor).

The term "therapeutically effective amount" is defined as an amount of an IL-21 composition or IL-21 composition in combination with a monoclonal antibody that results in a complete response, partial response, or stable disease with an increased time to progression over the median response duration for monoclonal antibody therapy without IL-21.

The term "tumor associated antigen" refers a peptide or polypeptide or peptide complex that has a different expression profile from antigen found on a non-tumor cells. For example, a non-tumor antigen may be expressed in higher frequency or density by tumor cells than by non-tumor cells. A tumor antigen may differ from a non-tumor antigen structurally, for example, the antigen could be expressed as a truncated polypeptide, have some mutation in the amino acid sequence or polynucleotide sequence encoding the antigen, be misfolded, or improperly modified post-translationally. Similar to antigens that are present on normal, non-tumor cells in the host organism allow the tumor cells to escape the host's immunological surveillance mechanisms.

Molecular weights and lengths of polymers determined by imprecise analytical methods (e.g., gel electrophoresis) will be understood to be approximate values. When such a value is expressed as "about" X or "approximately" X, the stated value of X will be understood to be accurate to ±10%.

All references cited herein are incorporated by reference in their entirety.

The present invention is based upon the discovery that administration of IL-21 in combination with therapeutic monoclonal antibodies result in antitumor activity that is more potent than administration of monoclonal antibodies alone.

### A. Description of IL-21.

Human IL-21 (SEQ ID NO:1 and SEQ ID NO:2) was originally designated zalpha11 Ligand, and is described in commonly-owned U.S. Patent Nos. 6,307,024, and 6,686,178, which are incorporated herein by reference. The IL-21 receptor, (previously designated zalpha11) now designated IL-21R (SEQ ID NO:5 and SEQ ID NO:6), and heterodimeric receptor IL-21R/IL-2Rγ are described in commonly-owned WIPO Publication No.s WO 0/17235 and WO 01/77171, which are incorporated herein by reference. As described in these publications, IL-21 was isolated from a cDNA library generated from activated human peripheral blood cells (hPBCs), which were selected for CD3. CD3 is a cell surface marker unique to cells of lymphoid origin, particularly T cells.

The amino acid sequence for the IL-21R indicated that the encoded receptor belonged to the Class I cytokine receptor subfamily that includes, but is not limited to, the receptors for IL-2, IL-4, IL-7, IL-15, EPO, TPO, GM-CSF and G-CSF (for a review see, Cosman, "The Hematopoietin Receptor Superfamily" in Cytokine 5(2): 95-106, 1993). The IL-21 receptor has been identified on NK cells, T cells and B cell indicating IL-21 acts on hematopoietic lineage cells, in particular lymphoid progenitor cells and lymphoid cells. Other known four-helical-bundle cytokines that act on lymphoid cells include IL-2, IL-4, IL-7, and IL-15. For a review of four-helical-bundle cytokines, see, Nicola et al., Advances in Protein Chemistry 52:1-65, 1999 and Kelso, A., Immunol. Cell Biol. 76:300-317, 1998.

For IL-21, a secretory signal sequence is comprised of amino acid residues 1 (Met) to 29 (Ser), and a mature polypeptide is comprised of amino acid residues 30 (Gln) to 162 (Ser) (as shown in SEQ ID NO: 2). The corresponding polynucleotide sequence is shown in SEQ ID NO:1. Those skilled in the art will recognize that the sequence disclosed in SEQ ID NO: represents a single allele of human IL-21 and that allelic variation and alternative splicing are expected to occur.

The present invention also provides isolated IL-21 polypeptides that have a substantially similar sequence identity to the polypeptides of SEQ ID NO:2, or their orthologs. The term "substantially similar sequence identity" is used herein to denote polypeptides comprising at least 80%, at least 90%, at least 95%, or greater than 95% sequence identity to the sequences shown in SEQ ID NO:2, or their orthologs. The present invention also includes polypeptides that comprise an amino acid sequence having at least at least 80%, at least 90%, at least 95% or greater than 95% sequence identity to the sequence of amino acid residues 1 to 162 or 30 to 162 of SEQ ID NO:2. The present invention further includes nucleic acid molecules that encode such polypeptides. Methods for determining percent identity are known to those skilled in the art.

In general, when designing modifications to molecules or identifying specific fragments determination of structure will be accompanied by evaluating activity of modified molecules. For extensive discussion of modifications to the IL-21 polynucleotide and polypeptide, see, U.S. Patent Nos. 6,307,024, and 6,686,178 which are incorporated herein by reference.

The present invention also includes administration of molecules having the functional activity of IL-21. Thus, administration of functional fragments and functional modified polypeptides of IL-21 polypeptides and nucleic acid molecules encoding such functional fragments and modified polypeptides are encompassed by the present invention. A "functional" IL-21 or fragment thereof as defined herein is characterized by its proliferative or differentiating activity, by its ability to induce or inhibit specialized cell functions, in particular for immune effector cells, such as NK cells, T cells, B cells and dendritic cells. Functional IL-21 also includes the ability to exhibit anticancer and antiviral effects *in vitro* or *in vivo,* or by its ability to bind specifically to an anti-IL-21 antibody or IL-21 receptor (either soluble or immobilized).

A variety of polypeptide fusions (and related multimeric proteins comprising one or more polypeptide fusions) can also be used. For example, a IL-21 polypeptide can be prepared as a fusion to a dimerizing protein as disclosed in U.S. Patents Nos. 5,155,027 and 5,567,584. Preferred dimerizing proteins in this regard include immunoglobulin constant region domains. Immunoglobulin- IL-21 polypeptide fusions can be expressed in genetically engineered cells (to produce a variety of multimeric IL-21 analogs). Auxiliary domains can be fused to IL-21 polypeptides to target them to specific cells, tissues, or macromolecules. For example, a IL-21 polypeptide or protein could be targeted to a predetermined cell type by fusing a IL-21 polypeptide to a ligand or monoclonal antibody that specifically binds to a receptor on the surface of that target cell. In this way, polypeptides and proteins can be targeted for therapeutic or diagnostic purposes. A IL-21 polypeptide can be fused to two or more moieties, such as an affinity tag for purification and a targeting domain. Polypeptide fusions can also comprise one or more cleavage sites, particularly between domains. See, Tuan et al., Connective Tissue Research 34:1-9, 1996.

Regardless of the particular nucleotide sequence of a variant IL-21 polynucleotide, the polynucleotide encodes a polypeptide that is characterized by its proliferative or differentiating activity, its ability to induce or inhibit specialized cell functions, or by the ability to bind specifically to an anti-IL-21 antibody or IL-21 receptor. More specifically, variant IL-21 polynucleotides will encode polypeptides which exhibit at least 50%, and in certain embodiments, greater than 70%, 80% or 90%, of the activity of the polypeptide as shown in SEQ ID NO: 2.

For any IL-21 polypeptide, including variants and fusion proteins, one of ordinary skill in the art can readily generate a fully degenerate polynucleotide sequence encoding that variant using the genetic code and methods known in the art.

The IL-21 polypeptides used in the present invention can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, 2nd ed., Cold Spring Harbor Laboratory Press, Cold Spring Harbor, NY, 1989, and Ausubel et al., eds., Current Protocols in Molecular Biology, John Wiley and Sons, Inc., NY, 1987. Expression constructs and methods for producing IL-21 are described in U.S. Patent No. 6,686,178 and PCT US03/39764, incorporated herein by reference.

IL-21 conjugates used for therapy can comprise pharmaceutically acceptable water-soluble polymer moieties. Suitable water-soluble polymers include polyethylene glycol (PEG), monomethoxy-PEG, mono-(C1-C10)alkoxy-PEG, aryloxy-PEG, poly-(N-vinyl pyrrolidone)PEG, tresyl monomethoxy PEG, PEG propionaldehyde, *bis*-succinimidyl carbonate PEG, propylene glycol homopolymers, a polypropylene oxide/ethylene oxide co-polymer, polyoxyethylated polyols (*e.g*., glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. A IL-21 conjugate can also comprise a mixture of such water-soluble polymers.

### B. Use of IL-21 and Monoclonal Antibodies in Combination Therapy.

One of the mechanisms associated with the antitumor activity of monoclonal antibody therapy is antibody dependent cellular cytotoxicity (ADCC). In ADCC, monoclonal antibodies bind to a target cell (e.g. cancer cell) and specific effector cells expressing receptors for the monoclonal antibody (e.g. NK cells, monocytes and granulocytes) bind the monoclonal antibody/target cell complex resulting in target cell death. IL-21 enhances effector cell function, thereby increasing monoclonal antibody therapy efficacy. The dose and schedule of IL-21 administration in combination with MAbs is based on the ability of IL-21 to elevate parameters associated with differentation and functional activity of cell populations mediating ADCC, including but not limited to, NK cells, macrophages and neutrophils. These parameters can be evaluated using assays of NK, macrophage and neutrophil cell cytotoxicity, ADCC (NK cell fraction or total mononuclear cells, or effector molecules essential to the ability of cells to implement ADCC (e.g., FasL, granzymes and perforin). IL-21 also increases cytokine and chemokine production by NK cells when combined with MAb plus tumor cells (e.g. IFNγ). The importance of Kupffer cells for "clearance" of rituximab-coated B cells has also been demonstrated (Gong et al., J. Immunol. 174:817-826, 2005). Another mechanism associated with antitumor activity is phagocytosis of MAb-coated tumor cells. This is also Fc receptor-dependent and has been shown to influence B depletion by anti-CD20 antibody (Uchida et al. J. Exp. Med. 199(12):1659-69, 2004). The dose and schedule of the MAbs is based on pharmacokinetic and toxicokinetic properties ascribed to the specific antibody co-administered, and should optimize these effects, while minimizing any toxicity that may be associated with IL-21 administration.

Based on the results with rituximab and trastuzumab described in detail herein, other monoclonal antibodies that utilize immune effector cell-mediated mechanisms for antitumor activity will also be enhanced when IL-21 is used in combination with the antibody. Moreover, because IL-21 enhances immune effector cell-mediated antitumor activity, certain monoclonal antibodies that have had limited antitumor efficacy when used alone will be good candidates for combination therapy with IL-21.

Combination therapy with IL-21 and a monoclonal antibody may be indicated when a first line treatment has failed and may be considered as a second line treatment. However, based on the enhanced antitumor activity of IL-21 in combination with a monoclonal antibody, the present invention also provides using the combination as a first line treatment in patient populations that are newly diagnosed and have not been previously treated with anticancer agents "de novo patients" and patients that have not previously received any monoclonal antibody therapy "naïve patients."

IL-21 is also useful in combination therapy with monoclonal antibodies in the absence of any direct antibody mediated ADCC of tumor cells. Antibodies that block an inhibitory signal in the immune system can lead to augmented immune responses. Examples include (1) antibodies against molecules of the B7R family that have inhibitory function such as, cytotoxic T lymphocyte-associated antigen 4 (CTLA-4), programmed death-1 (PD-1), B and T lymphocyte attenuator (BTLA); (2) antibodies against inhibitory cytokines like IL-10, TGFβ; and (3) antibodies that deplete or inhibit functions of suppressive cells like anti-CD25 or CTLA-4 . For example, anti-CTLA4 mAbs in both mice and humans are thought to either suppress function of immune-suppressive regulatory T cells (Tregs) or inhibit the inhibitory signal transmitted through binding of CTLA-4 on T cells to B7-1 or B7-2 molecules on APCs or tumor cells. CTLA-4 is expressed transiently on the surface of activated T cells and constitutively expressed on Treg cells. Cross-linking CTLA-4 leads to an inhibitory signal on activated T cells, and antibodies against CTLA-4 block the inhibitory signal on T cells leading to sustained T cell activation (Phan et al., PNAS, 100:8372-8377, 2003.) In mouse models, anti-CTLA4 treatment leads to an increase in numbers of activated tumor-specific CD8 T cells and NK cells resulting in potent antitumor responses. The receptor for IL-21 (IL-21R) is expressed on these effector cells and IL-21 may augment their effector function further by activating these cells through the IL-21R. This can lead to more potent antitumor activity. Clinical trials where blocking antibodies against CTLA-4 are administered to patients are ongoing in melanoma, ovarian and prostate cancer. However, efficacy has been correlated to serious adverse events (see, US 2004/0241169), and combination therapy resulting in less toxic treatment would be advantageous.

Table 1 is a non-exclusive list of monoclonal antibodies approved or being tested for which combination therapy with IL-21 is possible.

**Table 1**

| Target | Drug Name | Clinical Indication | Company |
|---|---|---|---|
| IL-2Rα(CD25) | Zenapax | kidney transplant | Roche |
| IL-1R | AMG108 | osteoarthritis | Amgen |
| RANK-L | AMG162 | osteoporosis | Amgen |
| Blys | LympoSTAT-B | SLE, RA | HGS |
| CD40L (CD39) | initiatedAID | Celltech/IDEC | |
| TRAIL-R1 | HGS-ETR1 | cancers | HGS |
| TRAIL-R2 | HGS-ETR2 | solid tumors | HGS |
| CD30 | SGN30 | Hodgkins, NHL | Seattle Genetics |
| CD40 | SGN40 | MM | Seattle Genetics |
| HER2 | Herceptin | Breast cancer | Genentech |
| EGF-R | ABX-EGF | CRC, NSCLC, RCC | Abgenix |
| | EMD72000 | solid tumors | Merck |
| | MDX-214 | EGF-R-positive tumors | Medarex |
| | Erbitux | CRC | Imclone |
| VEGF-R | CDP791 | solid tumors | Celltech |
| PDGF-R | CDP860 | solid tumors | Celltech/ZymoGenetics |
| CD11a(αL) | Raptiva | psoriasis | Genentech |
| α4-integrin | Antegrin | CD, MS | PDL, Biogen-IDEC |
| a4β7 integrin | MLM02 | CD, UC | Millenium |
| a5β3 integrin | Vitaxin | psoriasis, prostate cancer | AME/Lilly |
| CD2 (LFA3/Fc) | Amevive | psoriasis | Biogen/IDEC |
| CD152 | CTLA-4/Ig | RA | Bristol Meyers |
| CD152 | CTLA-4 | cancers | Medarex |
| CD49a | Integrin α1 | RA/Lupus | Biogen/IDEC |
| CD49e | Integrin α5 | cancers | Protein Design Labs |
| MUC1 | | | Theragyn |
| MUC18 (TIM-like) | ABX-MA1 | melanoma | |
| TAG-72 Mucin | Anatumomab | cancers | |
| CD3 | Ecromeximab | melanoma | Kvowa Hakko |
| | TRX4 | typeI IDDM | TolerRx |
| | Nuvion | UC | PDL |
| | OrthoCloneOKT3 | organ transplant | Ortho biotech |
| CD4 | HuMax-CD4 | T-cell lymphoma | GenMab |
| CD19 | MT103 | NHL | Medimmune |
| CD64 (Fc GR1) | AntiCD64 | cancers | Medarex |
| SIGLECs: | | | |
| CD33 | MyloTarg | AML | Celltech/Wyeth |
| | ZAmyl | AML | Protein Design Labs |
| CD22 | lymphocide | NHL, AID | Immunomedics |
| CEA | CEA-Cide | cancers | Immunomedics |
| CD20 | Rituxan | NHL | Genentech |
| CD52 | Campath | MS, NHL, T-cell lymph | Genzyme, IDEX |
| CD44 | Bivatuzumab | cancers | Boehringer Ingelheim |
| CD23 (Fc Ep R) | IDEC152 | allerhic asthma, rhinitis | Biogen/IDEC |
| LRR: | | | |
| CD14 | ICOSIC14 | sepsis | ICOS |
| EpCAM | Panorex | colorectal cancer | Centocor |
| Lewis-Y-Ag | SGN15 | cancers | Seattle Genetics |
| CD80 | B7.1 | psoriasis/NHL | Biogen/IDEC |

### 1. IL-21 and Anti-CD20 Monoclonal Antibodies

CD20 is a human B lymphocyte restricted differentiation antigen and is expressed as B cell surface antigen Bp35, a 35 kD protein. CD20 is found on peripheral B cells and can be identified on maturing B cells until the plasma cell stage (Reff et al., Blood 83:435-445, 1994). Anti-CD20 monoclonal antibodies (MAbs) have been tested in the clinic, and at least one humanized anti-CD20 MAb, rituximab, has been approved for treatment of Non-Hodgkins lymphoma (NHL). Rituximab (RITUXAN®) binds to lymphoma cells and can induce apoptosis directly *in vitro,* but is also capable of inducing a variety of effector mechanisms such as complement dependent cytotoxity and antibody dependent cell-mediated cytotoxicity (Shan et al., Blood 91:1644-1652, 1998). Rituximab is commonly used as a first line treatment for NHL (Maloney et al., Blood 90:2188-2195, 1997; U.S Patent No. 5,736,137).

Rituximab is a genetically engineered MAb with murine light- and heavy-chain variable regions and human gamma I heavy-chain and kappa light-chain constant regions (U.S. Patent 6,455,043). The chimeric antibody is composed of two heavy chains of 451 amino acids and two light chains of 213 amino acids and has an approximate molecular weight of 145 kD. In preclinical experiments, the antibody inhibited cell growth in the B-cell lines FL-18, Ramos, and Raji and induced apoptosis in the DHL-4 human B-cell lymphoma line in a dose-dependent manner (Demidem et al. Cancer Biotherapy & Radiopharmaceuticals 12:177-186, 1997). The MAb has been shown to have a relatively long half life in serum and the toxicity profile is relatively low.

However, a significant patient population is refractory or become resistant over time to treatment with anti-CD20 antibody, even when combined with other treatments such as bone marrow or stem cell transplantation, radiotherapy and chemotherapy. These patients generally do not exhibit appreciable tumor remission or regression after administration of anti-CD20 antibodies, and would benefit from new therapies which would enhance responsiveness to the antibodies. Moreover, enhanced antitumor activity will also benefit patient populations that are newly diagnosed and had not been previously treated with anticancer agents "de novo patients" and patients that have not previously received any monoclonal antibody therapy "naïve patients."

As stated previously, IL-21 has been shown to expand NK cells numbers and to potentiate the cytotoxic effects of NK cells and T cells. Moreover, receptors for IL-21 have been identified on monocytes, dendritic cells, B cells, T cells and NK cells (Parrish-Novak et al., J. Leuk. Biol. 72:856-863, 2002). Additional evidence has demonstrated that IL-21 affects proliferation and/or differentiation of T cells and B cells *in vivo.* Many human B cell tumor lines can be engrafted into SCID mice and grow in a localized or disseminated manner. In these models measurement of tumor growth or survival time of the host mouse provides a means for evaluating potential therapeutic efficacy against B cell cancers (Bonnefoix et al., Leukemia and Lymphoma 25:169-178, 1997).

When antibodies mediate an antitumor effect through ADCC by immune-based cells (including NK cells, macrophages and neutrophils) cancer cells that are bound by the antibody complex are killed by immune effector cells. IL-21 can be used to enhance the effectiveness of antibody therapy due in part to its immunomodulatory activity. Combination therapy with rituximab and a cytokine has been investigated using IL-2, IL-12, or IFN-α for the treatment of Hodgkin's and Non-Hodgkin's lymphoma (Keilholz et al., Leuk. Lymphoma 35:641-2,. 1999; Ansell et al., Blood 99:67-74, 2002; Carson et al., Eur. J. Immunol. 31:3016-25, 2001; and Sacchi et al., Haematologica 86:951-8., 2001).

Based on the ability of IL-21 to activate and differentiate effectors of ADCC, especially NK cells, *in vitro* and *in vivo* studies were performed that combined IL-21 with antibodies and assessed cytokine production, cytotoxicity and tumor clearance. The *in vitro* studies assayed cytokine production and tumor cell lysis by human NK cells after exposure to IL-21 and antibody. For example, tumor cell lysis can be evaluated using NK cells isolated from peripheral blood leukocytes. Human B cell lymphoma cell lines, such as DOHH2, Raji or Ramos, are loaded with calcein-AM or ⁵¹Cr, exposed to IL-21 for 1-7 days, and NK cell-mediated cell lysis is measured. Another assay measures cytokine production. Typically in these assays, purified NK cells are exposed to IL-21 and cultured *in vitro* with IgG adhered to the plates. The presence of such cytokines as INF-γ, TNF-α and IL-10 is measured. Detailed description of these types of assays can be found in the Examples section. *In vivo* studies monitoring survival of the mice after tumor challenge are taught herein. Other possible endpoints for *in vivo* studies can include weight loss, reduction in tumor mass or hindlimb paralysis (HLP). As shown in detail in the Examples section, the results of these experiments demonstrated that antitumor activity against CD20+ B cell tumors was significantly greater for the combination of rituximab and IL-21 than for either rituximab or IL-21 alone. Further experiments in additional animal models, including primates provide additional evidence for IL-21 enhancement of rituximab-mediated efficacy and are the basis for testing the combination in lymphoma patients.

Lymphocytes, which include B cells, T cells, NK cells and dendritic cells and their progenitors, have a life cycle that involves migration to and from various lymphoid and non-lymphoid tissues. All lymphocytes are believed to mature from a multipotent lymphoid progenitor residing in bone marrow. Naive lymphocytes cycle between blood and secondary lymphoid tissues until the cells die or are activated by antigen. When B or T cell lymphocytes are activated by antigen, the activated cells recirculate to the blood. There is evidence to suggest that chemokines play an important role in trafficking of lymphocytes. Expression of specific chemokines, such as CXCR3, are thought to promote trafficking of malignant B cells from one site to another, playing a role in the migration of B cell lymphomas to peripheral blood, lymph nodes, bone marrow and other organs (Trentin et al., J. of Clinical Invest. 104:115-121, 1999.) Rituximab has been shown to deplete B cells present in the peripheral blood and peripheral lymph nodes (Reff et al. Blood 83:435-445, 1994), and administration of an agent that drives CD20+ cells into these tissues would provide a mechanism to make previously inaccessible malignant cells more susceptible to rituximab-mediated killing. IL-21 has been shown to have both direct and indirect effects on B cells (Parrish-Novak et al., J. Leukoc. Biol. 72:856-863, 2002; Mehta et al., J. Immunol 170:4111-4118, 2003; Ozaki et al., J. Immunol. 173:5361-5371, 2004.) and is known to affect the maturation process in certain immune cells (Sivakumar et al., Immunol. 112:177-182, 2004.)

Experiments disclosed herein describe the present inventors discovery that administration of IL-21 initially reduced circulating B cells, T cells and NK cells, followed by a sustained increase and resolution prior to the next dosing cycle. The rapid reversal of lymphopenia and lymphoid follicle depeletion can be understood as transient margination of activated lymphocytes combined with increased recirculation from lymphoid tissues to blood. The increase in peripheral B cells was mitigated when IL-21 and rituximab were administered, and consistently lower B cell nadir was observed than was seen when either IL-21 or rituximab were administered alone. Thus, IL-21 enhances the potential for B cell depetion by rituximab, promoting recirculation of B cells that are suspectible to depletion. Moreover, administration of IL-21 resulted in enhanced ADCC activity, with increased numbers of NK cells and phagocytic cells expressing FcγRI and FcγRIII present when ADCC assays were performed.

Neutrophils have been shown to be important for the antitumor activity of rituximab in xenogeneic B lymphoma models (Hernandez-Ilizaliturri Clin. Cancer Res. 9(16 Pt. 1):5866-73, 2003). The role of granulocytes in the antitumor activity of mIL-21+rituximab is shown by depleting with an anti-GR-1 MAb. Groups of granulocyte-depleted and non-depleted SCID mice were challenged with Raji cells and then treated with rituximab alone or rituximab plus mIL-21 as described in Example 10. Granulocyte depletion reduced the survival of SCID mice treated with rituximab alone and with rituximab plus mIL-21. Comparing groups treated with combination therapy the fraction surviving after 125 days was reduced from 0.67 to 0.0 for the granulocyte-depleted animals. However, granulocyte depletion did not totally eliminate the survival benefit of IL-21 plus rituximab since a significant delay in the mean time to death (TTD) versus the vehicle control group is evident.

Macrophages have recently been shown to express IL-21 receptors (Pelletier et al. J. Immunol.173(12):7521-30, 2004) and to play a role in B cell depletion by anti-CD20 Mabs (Uchida et al. J. Exp. Med. 199(12):1659-69, 2004). Macrophages were depleted in SCID mice using clodronate liposomes and IL-21 plus rituximab was tested in the disseminated Raji lymphoma model. Depletion with clodronate liposomes eliminated 95% of F4/80⁺ cells in the liver and 90% of F4/80⁺ cells in the red pulp of the spleen. Macrophages were depleted three days after injecting Raji cells and macrophage depletion was maintained until at least 27 days following tumor cell injection by repeated clodronate liposome injections. Macrophage depletion also reduced the efficacy of mIL-21 plus rituximab. Mean TTD was reduced significantly for clodronate liposome treated groups. Also, there was a dramatic drop in the median survival time for macrophage-depleted SCID mice treated with rituximab alone as compared to the corresponding group of non-depleted mice.

Depletion of neutrophils with anti-Gr-1 dramatically reduced the efficacy of rituximab alone as reported by others (Hernandez-Ilizaliturri, ibid. 2003) and experiments showed it reduced the fraction of mice surviving after treatment with IL-21 plus rituximab from 0.67 to 0.0. IL-21 may be acting directly to affect mouse neutrophils that in turn may phagocytose tumor cells, effect ADCC or produce cytotoxic oxygen intermediates. But the direct action of IL-21 on neutrophils is not supported by studies of human neutrophils (Pelletier, ibid.) where IL-21Rα was not detected and IL-21 did not modulate neutrophil responses including superoxide production, phagocytosis, chemotaxis and cytokine production. Instead these authors found that IL-21 induced IL-8 production by human macrophages that may lead to neutrophil chemotaxis and activation. However, when experiments were performed supporting the present invention macrophage depletion using clodronate liposomes resulted in only a partial loss of the synergistic antitumor activity displayed by IL-21 and rituximab. These results suggested that in SCID mice both neutrophils and macrophages play a role in prolonging survival with combination therapy. Recent studies (Uchida et al., ibid.) of normal B cell depletion with anti-CD20 MAbs also show that mouse macrophages are the major effector cell required and that NK cells are not essential, however, neutrophils were not investigated in that study.

These findings demonstrate that IL-21 in combination with rituximab has synergistic antitumor activity in xenogeneic B lymphoma models, and that innate immune effector cells help mediate the synergistic effects of IL-21, and rituximab. These results suggest that in SCID mice both neutrophils and macrophages play a role in prolonging survival with combination therapy. IL-21 promotes the antitumor activity of rituximab on NHL and the action of IL-21 on macrophage, NK cells, T cells and lymphoma tumors themselves improves the response to rituximab therapy.

The present invention therefore provides a method of treating patients with lymphoma by administering IL-21 in combination with rituximab in patients where liberation of malignant cells from tissues is required for rituximab-mediated antitumor activity. Furthermore, dosing regimens that maintain IL-21 levels while rituximab is present in the patient's peripheral blood will be advantageous and are included in the present invention. In certain embodiments, the present invention provides a method of treating lymphoma in a patient in need thereof comprising administering IL-21 during the treatment period where rituximab is determined to be present in the patient's peripheral blood. In other embodiments, the present invention provides a method of treating lymphoma in a patient in need thereof comprising administering IL-21 one to three times weekly while the patient is receiving rituximab therapy.

The classification of Non-Hodgkin's lymphomas most commonly used is the REAL classification system (Ottensmeier, Chemico-Biological Interactions 135-136:653-664, 2001.) Specific immunological markers have been identified for classifications of lymphomas. For example, follicular lymphoma markers include CD20+, CD3-, CD10+, CD5-; Small lymphocytic lymphoma markers include CD20+, CD3-, CD10-, CD5+, CD23+; marginal zone B cell lymphoma markers include CD20+, CD3-, CD10-, CD23-; diffuse large B cell lymphoma markers include CD20+, CD3-; mantle cell lymphoma markers include CD20+, CD3-, CD10-, CD5+, CD23+; peripheral T cell lymphoma markers include CD20-, CD3+; primary mediastinal large B cell lymphoma markers include CD20+, CD3-, lymphoblastic lymphoma markers include CD20-, CD3+, Tdt+, and Burkitt's lymphoma markers include CD20+, CD3-, CD10+, CD5- (Decision Resourses, Non-Hodgkins Lymphoma, Waltham, MA., Feb. 2002).

Clinical classification of Non Hodgkins lymphoma (NHL) by the International Working Formulation breaks down disease into subtypes: (1) low grade (indolent) disease which includes small lymphocytic, consistent with chronic lymphocytic leukemia (SC); follicular, predominantly small cleaved cell (FSC); follicular, mixed small cleaved and large cell (FM); (2) intermediate grade disease which includes follicular, predominantly large cell (FL); diffuse, small cleaved cell (DSC); diffuse mixed, small and large cell (DM); diffuse, large cleaved or noncleaved cell (DL); and (3) high grade disease which includes immunoblastic, large cell (IBL); lymphoblastic, convoluted or nonconvoluted cell (LL); and small noncleaved cell, Burkitt's or non-Burkitts (SNC; (The Non-Hodgkin's Lymphoma Pathologic Classification Project, Cancer 49 (10):2112-35, 1982). The Ann Arbor Staging system is commonly used to stage patients with NHL. Stage I means involvement of a single lymph node region or localized involvement of a single extralymphatic organ or site. Stage II means involvement of two or more lymph node regions on the same side of the diaphragm or localized involvement of an extranoldal site or organ and one or more lymph node regions on the same side of the diaphragm. Stage III means involvement of lymph node regions on both sides of the diaphragm, possibly accompanied by localized involvement of an extranodal organ or site. Stage IV means diffuse or disseminated involvement of one or more distant extranodal organs with or without associated lymph node involvement ("Lymphoid neoplasms." In: American Joint Committee on Cancer.: AJCC Cancer Staging Manual. 6th ed. New York, NY: Springer, 2002, pp 393-406). Rituximab has been shown effective in treating indolent and follicular lymphomas (Boye et al., Annals of Oncol. 14:520-535, 2003).

The activity of IL-21 in combination with anti-CD20 antibodies on growth and dissemination of tumor cells derived from human hematologic malignancies can be measured *in vivo.* Several mouse models have been developed in which human tumor cells are implanted into immunodeficient mice (collectively referred to as xenograft models); see, for example, Cattan et al., Leuk. Res. 18:513-22, 1994 and Flavell, Hematological Oncology 14:67-82, 1996. The characteristics of the disease model vary with the type and quantity of cells delivered to the mouse, and several disease models are known in the art. For example, human B cell lymphomas (e.g. RL, Raji, TU2C) grown and disseminated in SCID mice can be treated with MAbs and IL-21 to prolong survival using models known to those skilled in the art. For exemplary models, see, Funakoshi et al., J. Immunotherapy 19:93-101, 1996; Funakoshi et al., Blood 83:2787-94, 1994; Cattan et al., Leukemia Res. 18:513-522, 1994. Alternatively, mouse B cell lymphoma cells lines (A20, BCL, A31) can be implanted and treated with MAbs and IL-21 to prolong survival (French et al., Nat. Medicine 5:548-553, 1999; Tutt et al., J. Immunol. 161:3176-3183, 1998). In one model, tumor cells (e.g. Raji cells (ATCC No. CTL-86)) are passaged in culture and about 1X10⁶ cells injected intravenously into severe combined immune deficient (SCID) mice. Such tumor cells proliferate rapidly within the animal and can be found circulating in the blood and populating numerous organ systems. Therapies designed to kill or reduce the growth of tumor cells using IL-21 and anti-CD20 MAbs are tested by administration of IL-21 and MAb to mice bearing the tumor cells. Efficacy of treatment is measured and statistically evaluated as increased survival within the treated population over time. Tumor burden may also be monitored over time using well-known methods such as flow cytometry (or PCR) to quantitate the number of tumor cells present in a sample of peripheral blood.

Animal models that can be used to demonstrate efficacy of combination therapy using IL-21 and anti-CD20 MAbs include non-human primate models of B-cell depletion. For example, by treating Cynomolgus monkeys with either vehicle, 0.05 mg/kg or 10.0 mg/kg rituximab various B cell CD20, CD40 and CD21 populations were identified as useful in studying anti-CD20 therapeutics (Vugmeyster et al., Internat. Immunol. 3:1477-1481, 2003.

Rituximab therapy for indolent disease generally consists of four once weekly infusions of 375 mg/m². Initial infusion rate is 50 mg/hr, and is escalated to a maximum of 400 mg/hr in 50 mg increments every 30 minutes (McLaughlin et al., Clinical Oncol. 16:2825-2833, 1998). However, extended treatment of eight weeks has shown some efficacy in treatment for refractory or relapsed low-grade or follicular NHL (Piro et al., Ann. Oncol. 10:619-621, 1999).

Establishing the optimal dose level and scheduling for IL-21 and anti-CD20 MAb combination therapy is done using multiple means, including the pharmacokinetics and pharmacodynamics of the combination, the sensitivity of human B-cell lymphoma lines and primary lymphoma specimens to a combination of IL-21 and anti-CD20 MAbs *in vitro,* effective doses in animal models and the toxicity of the combination. Direct pharmacokinetic measurements can be done in primates. In addition IL-21 and anti-CD20 MAbs stimulate a variety of responses in normal lymphocytes, such that clinical efficacy may be measured in normal animal models. Moreover, surrogate markers can be employed to measure the biological activity of the combination of IL-21 and anti-CD20 MAb on effector cells in patients. Surrogate markers include, but are not limited to, significant decreases in B cell populations, increases in NK cell population, monocyte/macrophage activation, FcRIII increases, increases in cytotoxicity of NK or T cells on CD20+ cells in the presence of anti-CD20 antibody. Surrogates are valuable as indicators of efficacy because therapeutical tumor responses such as an increase in survival can require months to years to determine.

Treatment of lymphoma, such as NHL or chronic lymphoblastic leukemia (CLL), using a combination of IL-21 and rituximab is demonstrated using clinical studies where safety and efficacy are investigated. Initially, safety is demonstrated in a phase I study which is an open-label study of doses which escalate until either a maximally tolerated dose (MTD) or optimal immunologic dose is identified. An optimal immunologic dose is identified as the dose IL-21 or IL-21 in combination with a monoclonal antibody that achieves the optimal immunological response. An optimal immunological response refers to a change in an immunological response after administration of IL-21 or the IL-21 + MAb combination over that seen when the MAb alone is administered and can be measured as described herein.

Participants in an initial phase I study are subjects with relapsed or refractory CD20+ NHL. Dose escalation is evaluated in cohorts of 3 to 6 subjects in a standard 3 plus 3 dose escalation scheme. Cohorts of 3 subjects are evaluated for any dose-limiting toxicity (DLT) occurring by the end of the fourth week. In the absence of DLT, dose escalation occurs. If 1 of 3 subjects has an observed dose-limiting toxicity, an additional 3 subjects are enrolled at that dose level. If > 1 subjects in a given cohort experience dose-limiting toxicity, then dose de-escalation occurs and 3 subjects are treated at an intermediate dose to be specified by a Safety Monitoring Committee (SMC). The dose would be between the dose that elicited DLT and the next lower dose. If 0 out of 3 subjects experience a DLT at the intermediate dose, then enrollment is halted and the intermediate dose would be declared MTD. If ≥ 1 out of 3 subject experiences DLT at the intermediate dose, then enrollment is halted and the dose level below that would be declared MTD.

Subjects are administered rituximab, intravenously (IV) at 375 mg/m², once weekly and administered for either four or eight weeks consecutively. IL-21 is given by injection, either by IV, or intramuscular (IM) or subcutaneous (SC.) routes of administration. The first cohort is given at least 1 µg/kg and doses escalate to MTD or an optimal immunological dose, in a step-wise fashion, for example, increasing from 3-10, 10-100, 100-300, 300-500, 500-900 and up to 1000 µg/kg from once to five times weekly. The present invention provides for IL-21 compositions wherein each dose is in a range of about 1 µg/kg to 1000 µg/kg. In certain embodiments, the IL-21 dose is in the range of 10 to 300 µg/kg.

Tumor response is used to assess primary clinical activity. To assess antitumor response, restaging occurs at weeks 4, 8, and 12 using, for example, the International Workshop to Standardize Response Criteria for Non-Hodgkin's Lymphomas (Cheson et al, J. Clin. Oncol. 17:1244-1253, 1999). Pharmacodynamic markers of IL-21 are used as secondary indicators of clinical activity.

Adverse events and standard safety laboratory evaluations are used to evaluate safety. Analysis of serum for antibodies to IL-21 will be performed to assess immunogenicity.

Dose limiting toxicity is defined using the Common Terminology Criteria for Adverse Events (CTCAE) Version 3, dated December 12, 2003, as any of the following:
- Any Grade 4 or 5 adverse event probably or definitely related to study agent
- Non-hematologic Grade 3 adverse events probably or definitely related to study agent EXCEPT those related to lymphopenia of ≤ 7 days duration, tumor flare, fever, malaise, or non-life threatening laboratory abnormalities of Grade 3 that are clinically insignificant.

Efficacy and safety are further evaluated in phase II and phase III clinical studies. In these studies additional pharmacokinetics, pharmcodynamics, pharmacogenetics, pharmacogenomics, immunogenicity may be characterized. A primary endpoint is identified according to the International Workshop to Standardize Response Criteria for Non-Hodgkin's Lymphomas (Cheson et al., *ibid*.) and in accord with regulatory guidance. Secondary endpoints may include incidence and severity of adverse events, time to progression, time to relapse for complete responders, overall survival, and incidence of any antibody development to IL-21. The study can be a randomized, two-arm study comparing rituximab monotherapy with rituximab combined with IL-21 in patients who cannot tolerate or choose not to receive chemotherapy. Subjects will receive rituximab, administered IV at 375 mg/m², once weekly and administered for either four or eight weeks consecutively. IL-21 is administered IV or SC as a sequential infusion on the same day and up to five days consecutively, and IL-21 doses will be in the range of 1-3, 3-10, 10-100, 100-300, 300-500, 500-900 and up to 1000 µg/kg. Alternatively, a randomized three arm study maybe initiated to evaluate the safety and efficacy of IL-21 in combination with rituximab vs. IL-21 alone vs. rituximab alone using similar criteria for trial design. Clinical trial design is well known to those skilled in the art and guidelines provided by the Food and Drug Administration (FDA), for example at the FDA Oncology Tools website.

IL-21 and IL-15 or IL-2 exhibit synergy in their effects on NK-cells *in vitro* with respect to IFN-γ production cytotoxicity and proliferation (Parrish-Novak et al., J. Leuk. Biol. 72:856-863, 2002). However, high dose IL-2 therapy is highly toxic and requires extensive hospitalization. Many low dose regimens of IL-2 have been tested, and found to be better tolerated, but with little evidence of antitumor efficacy (Atkins, Semin. Oncol. 29 (3 Suppl. 7):12, 2002). IL-2 and rituximab combination therapy is described in WO 03/049694, where IL-2 is administered at higher "loading" dose, followed by one or more lower "maintenance" doses. The need to continue dosing of IL-2 is based on maintaining NK cell levels at higher than normal levels, but due to the toxicity of IL-2, a rest period in which IL-2 is not administered may be required. Administration of the combination of IL-2 and IL-21 in addition to anti-CD20 MAbs will maintain NK cells and permit lower or less frequent dosing with IL-2. Certain side effects seen with high dose IL-2 have not been demonstrated when IL-21 has been administered. For example, when IL-21 was administered to mice at the dose and schedule of IL-2 reported to cause vascular leak syndrome in mice, vascular leak syndrome was not present. The results clearly show that IL-21 does not elicit the cytokine release and vasculitis associated with an equivalent mass-based dose of rIL-2 in mice (Heipel et al., Blood 102 (11):No. 2845, 2003). The combination of low dose EL-2 with IL-21 and anti-CD20 MAbs therefore may be clinically useful by augmenting the immune system stimulation of low dose IL-2 without certain side effects caused by higher IL-2 doses.

Administration of IL-21 in combination with anti-CD20 MAbs, such as rituximab, using the methods of the present invention will result in an antitumor effect, also referred to as tumor response. Standardized guidelines for evaluation of response to therapy for NHL are known to those skilled in the art. An explemary set of uniform criteria is described in Cheson et al., J. of Clinical Oncol. 17:1244-1253, 1999. The International Working Group set forth recommendations and definitions of response measurements. Table 2 summarizes the response criteria.

**Table 2**

| Response Category | Physical Examination | Lymph Nodes | Lymph Node Masses | Bone Marrow |
|---|---|---|---|---|
| Complete Response (CR) | Normal | Normal | Normal | Normal |
| Complete Response unconfirmed (CRu) | Normal | Normal | Normal | Indeterminate |
| Partial Response (PR) | Normal | Normal | Normal | Positive |
| PR | Normal | ≥50% decrease | ≥50% decrease | Irrelevant |
| PR | Decrease in liver/spleen | ≥50% decrease | >50% decrease | Irrelevant |
| Relapse/ Progression | Enlarging liver/spleen; new sites | New or increased | New or increased | Reappearance |

Surrogate markers may be used to indicate enhanced antitumor activity as well. For example, a change in serum enzymes and biopsy can demonstrate a decrease in tumor burden.

One measure of bioactivity that can be used as a surrogate for antitumor effect is maintenance of NK cell levels at a level that enhances the antitumor effect of an anti-CD20 MAb (Friedberg et al., Br. J. Hematol. 117:828-834, 2002). Another surrogate is T cell number increases (Parrish-Novak et al., ibid. 2002). In particular, increased cell number for a subset of T cells has been correlated with increased cytotoxic activity or antitumor effect. Another measure of bioactivity that can be used as a surrogate for antitumor effect is depletion of B cells (Reff et al., Blood 83:435-445, 1994).

### 2. Use of IL-21 and anti-Her-2/neu Monoclonal Antibodies in Combination Therapy

Her-2/neu gene product is a 185 kDa phosphosglycoprotein that is related to epidermal growth factor receptor. Her-2/neu functions as a growth factor receptor and is often expressed by tumors such as breast cancer, ovarian cancer and lung cancer. Her-2/neu receptor is overexpressed in 25-30% of human breast cancers (Slamon et al. Science 235:177-182, 1987; Slamon et al., Science 244:707-712, 1989) and is associated with a poor prognosis in these patients.

There are a number of monoclonal antibodies that target Her-2/neu, but HERCEPTIN®, the trade name for trastuzumab (Genentech, Inc., San Francisco, CA) is presently the only approved therapeutic for treatment of Her-2/neu positive cancer patients. Small amounts of Her-2/neu can be found on many normal cell types, and cancer cells have altered expression leading to overexpression, increased cell proliferation and differentiation associated with the cancer cell phenotype. However, successful treatment with trastuzumab requires that Her-2/neu expression be highly overexpressed. Her-2/neu expression levels can be determined using biopsy samples that are fixed and immunohistologically stained. These types of assays are well known in the art and include immunohistochemical assessment using the 4D5 monoclonal antibody (LabCorp, Research Triangle Park, NC), HerceptTest^{®} (DAKO, Glostrup, Denmark) and Vysis PathVysion™ HER-2 DNA Probe Kit (Fujisawa Healthcare, Inc., North Deerfield, IL). Her-2/neu levels are generally 0 (normal) to 3+, and trastuzumab therapy has been shown to be efficacious in patients with 2+ or greater expression levels.

TL-21 has been demonstrated (e.g., Example 6) to promote lytic activity in immune effector T cells and NK cells in both *in vitro* and *in vivo* models with human breast cancer cell lines expressing either high levels or lower levels of Her-2/neu receptor. IL-21 mediated enhanced effector function results in trastuzumab therapy being efficacious even where cancer cells express lower levels of Her-2/neu receptor. For example, patients with 1+ or 2+ overexpression levels treated with IL-21 and trastuzumab will be candidates for treatment, providing valuable therapy for previously untreated patient populations. Mice bearing Her-2/neu expressing murine carcinomas can used to test IL-21 in combination with antiHer-2/neu MAbs (Penichet, et al., Lab Anim. Sci. 49:179-188, 1999).

While each protocol may define tumor response assessments differently, exemplary guidelines can be found in Clinical Research Associates Manual, Southwest Oncology Group, CRAB, Seattle, WA, October 6, 1998, updated August 1999. According to the CRA Manual (see, chapter 7 "Response Accessment"), tumor response means a reduction or elimination of all measurable lesions or metastases. Disease is generally considered measurable if it comprises bidimensionally measurable lesions with clearly defined margins by medical photograph or X-ray, computerized axial tomography (CT), magnetic resonance imaging (MRI), or palpation. Evaluable disease means the disease comprises unidimensionally measurable lesions, masses with margins not clearly defined, lesion with both diameters less than 0.5 cm, lesions on scan with either diameter smaller than the distance between cuts, palpable lesions with diameter less than 2 cm, or bone disease. Non-evaluable disease includes pleural effusions, ascites, and disease documented by indirect evidence. Previously radiated lesions which have not progressed are also generally considered non-evaluable.

The criteria for objective status are required for protocols to assess solid tumor response. A representative criteria includes the following: (1) Complete Response (CR) defined as complete disappearance of all measurable and evaluable disease. No new lesions. No disease related symptoms. No evidence of non-evaluable disease; (2) Partial Response (PR) defined as greater than or equal to 50% decrease from baseline in the sum of products of perpendicular diameters of all measureable lesions. No progression of evaluable disease. No new lesions. Applies to patients with at least one measurable lesion; (3) Progression defined as 50% or an increase of 10 cm² in the sum of products of measurable lesions over the smallest sum observed using same techniques as baseline, or clear worsening of any evaluable disease, or reappearance of any lesion which had disappeared, or appearance of any new lesion, or failure to return for evaluation due to death or deteriorating condition (unless unrelated to this cancer); (4) Stable or No Response defined as not qualifying for CR, PR, or Progression. (See, Clinical Research Associates Manual, supra.)

The invention is further illustrated by the following non-limiting examples.

### EXAMPLES

### Example 1

### IL-21 Enhances Antibody-Dependent NK Cell Activity

### A.

Peripheral blood was obtained and mononuclear cells (MNC's) were prepared by ficoll centrifugation. Natural killer (NK) cells were purified from the MNC population by negative enrichment, utilizing the StemSep™ Human NK Cell Stem Cell Technologies (Vancouver, British Columbia) human NK cell negative enrichment kit. Briefly, MNC's were labeled with lineage specific antibodies (excluding the NK lineage) and were in turn magnetically labeled. The labeled MNC's were then run over a magnetic column where the labeled cells were retained and the non-labeled NK cells flowed through.

NK cells were plated at a density of 5x10⁵ cells/mL and cultured for 3 days in αMEM/10% autologous serum/50 µM β-mercaptoethanol, with 0, 1, 10, or 100 ng/mL hIL-21 (A794F) or 10 ng/mL IL-12 (positive control), all in the presence or absence of Fc stimulation. Fc stimulation was provided by plating 100 µg/mL hIgG in PBS onto plastic at 37°C for 1 hour, then the PBS/antibody solution was removed, and NK's were cultured on that surface. After the three-day culture period, supernatants were collected. IFN-γ in the supernatants was quantified using the BD OptEIA human IFN-γ ELISA kit (BD Biosciences, San Jose, CA). Results were plotted in bar chart form, expressing ng/mL IFN-γ per sample.

In the presence of Fc stimulation, IL-21 caused a dose dependent increase in IFN-γ production. At the maximum dose of IL-21 tested in this experiment (100 ng/mL) there was an increase of roughly 18 fold over background. In the absence of Fc stimulation, there was no increase in IFN-γ production in the presence of IL-21.

### B.

Peripheral blood leukocytes were obtained by leukopheresis from a donor program. Mononuclear cells (MNC's) were prepared from apheresed blood by ficoll centrifugation. Natural killer (NK) cells were purified from the MNC population by negative enrichment, utilizing the Stem Cell Technologies human NK cell negative enrichment kit. Briefly, MNC's were labeled with lineage specific antibodies (excluding the NK lineage) and were in turn magnetically labeled. The labeled MNC's were then run over a magnetic column where the labeled cells were retained and the non-labeled NK cells flowed through.

NK cells were plated at a density of 1x10⁶/mL and cultured for 1, 2, 3, 4, 6, or 7 days in αMEM/10% heat-inactivated human AB serum/50µM beta mercaptoethanol/ITS (Invitrogen GibcoBRL, Carlsbad, CA)/150 µg/ml supplemental transferrin/5 mg/mL BSA, in the presence or absence of 0.2, 1, 5, 25, or 100 ng/mL human IL-21. At the end of each culture period, NK cells were harvested, washed, counted, and placed into an antibody dependent cellular cytotoxicity cytolytic (ADCC) assay, utilizing a lymphoma cell line (Ramos, CRL 1596, American Type Culture Collection, Manassas, VA) as the cytolytic target. Target cells were labeled prior to the assay by incubating for 60 minutes at 37C in Hanks Buffered Saline Solution (without Ca or Mg) with 5% FBS (HBSSF) and 10 µM calcein AM (Molecular Probes, cat no C 1430). The target cells take up the fluorescent dye (calcein AM) and cytoplasmically convert it into the active fluorochrome, which is only released from the cell upon lysis. Lysed cells release the fluorochrome into the supernatant, which is then harvested and the amount of fluorescence quantitated in a fluorometer. The percent cell lysis was calculated from the amount of fluorescence present in the supernatant after a 3-hour incubation in the presence or absence of varying amounts of NK cells (effectors). For the ADCC assay, targets were used with no added antibody, 1 µg/mL irrelevant IgG, or 1 µg/mL rituximab.

Two donors were tested. Donor A NK cells were cultured in 0, 1, 5, 25, or 100 ng/mL human IL-21, with time points on day 1, 2, 3, 4, and 7. Donor B NK cells were cultured in 0, 0.2, 1, 5, or 25 ng/mL human IL-21, with time points on days 1, 2, 3, 4, 6, and 7. In both donors there was an enhancement (3-10 fold) of cytolytic activity against target cells in the presence of rituximab, when compared to the irrelevant IgG control. This enhancement in cytolytic activity was further increased (2-10 fold) when the NK cells were cultured in the presence of IL-21 prior to the assay.

Donor A cultures showed no significant difference in the enhancement of ADCC among the doses of IL-21 tested (1, 5, 25, or 100 ng/mL) except on day 7, when the 1 ng/mL IL-21 NK culture had significantly less ADCC enhancement activity than the other doses. Donor B cultures showed no significant difference in the enhancement of ADCC among the doses of IL-21 tested (0.2, 1, 5, or 25 ng/mL) until day 4 (and continuing through the remaining time points) when the cultures containing 0.2 ng/mL IL-21 showed significantly less ADCC enhancement activity than the other IL-21 doses tested. Both donors showed an IL-21 ADCC enhancement at all time points tested, with the largest enhancement relative to the irrelevant IgG apparent on days 6 or 7.

### C.

Peripheral blood was obtained from a donor program as described in Example 1A. NK cells were plated at a density of 8.1-11 x 10⁵/mL and cultured for 3 days in αMEM/10% autologous serum/50 µM beta-mercaptoethanol, in the presence or absence of 20 ng/mL human IL-21. At the end of the culture period, NK cells were harvested, washed, counted, and placed into an antibody dependent cellular cytotoxicity cytolytic (ADCC) assay, utilizing the lymphoma cell line DOHH2 (Kluin-Nelemans, H.C. et al. Leukemia 5: 221-224, 1991; Drexler, H.G. et al., DSMZ Catalogue of Cell Lines, 7th edn, Braunschweig, Germany, 1999) as the cytolytic target. DOHH2 cells were labeled prior to the assay by incubating for 30 minutes in Hanks Buffered Saline Solution with 5% FBS (HBSSF) with 25 µM calcein AM (Molecular Probes). The targets take up the fluorescent dye (calcein AM) and cytoplasmically convert it into the active fluorochrome, which is only released from the cell upon lysis. Lysed cells release the fluorochrome into the supernatant, which is then harvested and the amount of fluorescence quantitated in a fluorometer. The % cell lysis was calculated from the amount of fluorescence present in the supernatant after a 3-hour incubation in the presence or absence of varying amounts of NK cells (effectors). For the ADCC assay, targets were used with no added antibody, 2 □g/mL irrelevant IgG, or 0.002, 0.02, 0.2, or 2 µg/mL rituximab.

Results were generated from two donors, and were expressed as effector:target (E:T) ratio vs. percent lysis. In both donors, there was a clear enhancement (6-11 fold at an E:T=3) of cytolytic activity against DOHH2 cells in the presence of 2 µg/mL rituximab, when compared to the no added antibody or irrelevant IgG control. The rituximab enhancement was the same at 2 µg/mL and 0.2 µg/mL, began to drop off at the highest E:T tested (4 or 6) at 0.02 µg/mL, and was clearly lower at all E:T's tested at 0.002 µg/mL. The enhancement in rituximab-dependent cytolytic activity was increased at all rituximab doses tested (1.5-3 fold over rituximab enhanced activity at an E:T=3) when the NK cells were cultured for 3 days in the presence of IL-21 prior to the cytolytic assay.

### Example 2

### IL-21 Upregulates Granzyme B Expression in Human NK Cells

Human NK cells were isolated from Ficoll-Paque purified mononuclear cells by negative selection using a magnetic bead separation kit. (Miltenyi Biotech, CA) Purified NK cells were then cultured for 48 hours in either medium alone or 20 ng/mL human IL-21. Cells were harvested, washed and then stained with surface markers. Following surface marker staining, cells were washed and then permeabilized with Cytofix/Cytoperm™ buffer (BD Biosciences, San Jose, CA) for 20 minutes. Cells were then stained with an APC-labeled anti-human Granzyme B or Isotype control antibody (Caltag, Burlingame, CA) in Perm/Wash buffer. Cells were washed and then read on a FACSCalibur™ flow cytometer. Data were analyzed using Cellquest™ software (BD Biosciences).

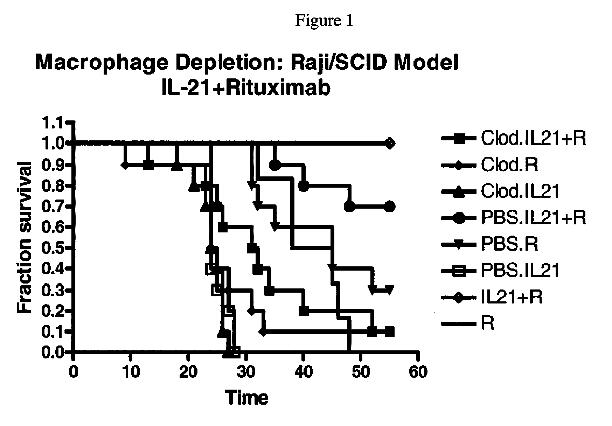

Figure 1 shows that incubating human NK's in the presence of IL-21 causes a large increase in Granzyme B expression, an important mediator of NK cell killing. This suggests that by upregulating Granzyme B, IL-21 enhances the ability of NK cells to kill their target cells.

### Example 3

### IL-21 + rituximab Increase Survival of Mice Injected with HS Sultan Lymphoma Cells

A study was done to evaluate whether tumor growth was delayed in CB-17 SCID mice injected with HS-Sultan cells treated the rituximab, mouse IL-21 (mIL-21) or a combination of mIL-21 and rituximab. The study was designed to characterize survival of HS-Sultan bearing mice in the various treatment groups.

The protocols were similar to those known in the art (see, Cattan et al. Leuk Res. 18 (7):513-522, 1994; Ozaki et al, Blood 90 (8):3179-86, 1997). CD17-SCID mice were either given 20 µg of rituximab (doesed every four days for a total of 5 injections), 100 µg of mIL-21 (dosed five days) or a combination of rituximab and mIL-21 via IP injections (dosed five times for each treatment).

Mice were monitored for moribund or non-survivable conditions such as paralysis or rapid weight loss. Body weights were collected twice a week during the term of the study. Survival time was recorded for all mice, and was compared between treatment groups by polotting Kaplan-Meier survival surves and computing log rank statistics (Statview, SAS Institute, Cary, NC).

The following groups were used:
Group 1 (n=10) 20 µg rituximab every 4 days for a total of 5 injections starting on day 1.
Group 2 (n=10) 20 µg rituximab every 4 days for a total of 5 injections starting on day 3.
Group 3 (n=10) 20 µg rituximab every 4 days for a total of 5 injections starting on day 6.
Group 4 (n=10) vehicle control (PBS) give IP days 1-5.
Group 5 (n=10) 100 µg mIL-21 IP daily for 5 days starting on day 1.
Group 6 (n=10) 100 µg mIL-21 for 5 days starting day 1 + 20 µg rituximab every 4 days for a total of 5 injections starting on day 3.

Mice (female, C.B-17 SCID, 9 weeks old; Harlan, Madison, WI) were divided into six groups. On day 0, HS-Sultan cells (ATCC No. CRL-1484) were harvested from culture and injected intravenously, via the tail vein, to all mice (1,000,000 cells per mouse). Mice were then treated with rituximab, mIL-21, or a combination of the two agents, using the doses and schedules described in the treatment group descriptions above. All treatments were administered by intraperitoneal injection in a volume of 0.1 mL.

In the groups of mice treated with rituximab, a significant survival benefit was observed when dosing was initiated on Day 1 or Day 3, but not on Day 6. Murine IL-21 alone provided no survival benefit to the tumor-bearing mice. Mice treated with a combination of mIL-21 (100 ug/day, day 1-5) and rituximab (20 ug/day, days 3, 7, 11, 15, 19) had a highly significant survival benefit (*P* < 0.0001 compared to vehicle control, *P* < 0.02 compared to rituximab starting on Day 3; Logrank test). On Day 120 of the study, cumulative survival in the mIL-21 + rituximab group was 70%, compared to 20% in the rituximab only group.

### Example 4

### IL-21 + rituximab Increase Survival of Mice Injected with Raji Tumor Cells

A study was done to evaluate whether tumor growth was delayed in CD-17 SCID mice injected with Raji cells treated with rituximab, mIL-21 or a combination of mIL-21 and rituximab. The study was designed to characterize survival of Raji bearing mice in the various treatment groups.

The protocol is described in Example 3.

The following groups were used:
Group 1 (n=8) vehicle control PBS by IP days 3-7
Group 2 (n=8) 100 µg mIL-21 IP daily for 5 days starting day 1.
Group 3 (n=8) 100 µg mIL-21 for 5 days starting day 3.
Group 4 (n=9) 20 µg rituximab every 4 days for a total of 5 injections starting on day 3.
Group 5 (n=9) 20 µg rituximab every 4 days for a total of 5 injections starting on day 5.
Group 6 (n=9) 100 µg mIL-21 IP daily for 5 days starting day 1 + 20 µg rituximab every 4 days for a total of 5 injections starting on day 3.
Group 7 (n=9) 100 µg mIL-21 IP daily for 5 days starting day 3 + 20 µg rituximab every 4 days for a total of 5 injections starting on day 5.

Mice (female, C.B-17 SCID, 9 weeks old; Harlan, Madison, WI) were divided into seven groups. On day 0, Raji cells (ATCC No. CCL-86) were harvested from culture and injected intravenously, via the tail vein, to all mice (1,000,000 cells per mouse). Mice were then treated with rituximab, mIL-21, or a combination of the two agents, using the doses and schedules described in the treatment group descriptions above. All treatments were administered by intraperitoneal injection in a volume of 0.1 mL.

In the groups of mice treated with rituximab, a significant survival benefit was observed when dosing was initiated on Day 3, or on Day 5. Murine IL-21 alone provided no survival benefit to the tumor-bearing mice. Mice treated with a combination of mIL-21 (100 ug/day, day 3-7) and rituximab (20 ug/day, days 5, 9, 13, 17, 21) had a highly significant survival benefit (*P* < 0.0001 compared to vehicle control, *P* < 0.03 compared to rituximab starting on Day 5; Logrank test). On Day 100 of the study, cumulative survival in the mIL-21 + rituximab group was 55%, compared to 10% in the rituximab only group.

### Example 5

### IL-21 + rituximab Studies In Non-human Primates

Rituximab and rIL-21 were co-administered intravenously to groups consisting of three male cynomolgus monkeys for three dosing periods consisting of one week per dose period. There was one week without dosing between the second and third week of dosing. Rituximab was dosed on the first day of each dosing period, and rIL-21 was dosed for three days beginning on the first day of each dosing period. Dosing exceptions were the control group dosed with control article 0.9% sodium chloride, Group 3 dosed with rituximab only, and Group 2 which received rIL-21 only. Group 5 was dosed with rIL-21 on the first day only of each dosing period, however the total weekly dose was equivalent to other groups receiving rIL-21. Group 7 was dosed with rIL-21 subcutaneously, rather than intravenously. Group 4 was not dosed during the third dosing period; the last dose was received on Day 10. The last dose for all other groups was on Day 24. The animals were dosed using intravenous injection into the cephalic, saphenous, or other suitable vein; subcutaneous injection into the intrascapular area or other suitable site.

**Table 3 Study Schedule and Groups**

| Group | Treatment | Route | Dose Levels (mg/kg) | Concentration (mg/mL) | Dose volume b (mL/kg) | Dose Days |
|---|---|---|---|---|---|---|
| 1 | Control Article | IV | 0.0 | 0.0 | 3.0 0.5 | 1, 8, 22 2, 3, 9, 10, 23, 24 |
| 2 | rIL-21 Control Article | IV | 0.5 0.0 | 1.0 0.0 | 0.5 2.5 | 1-3, 8-10, 22-24 1, 8, 22 |
| 3 | Control Article Rituxan^{c} | IV | 0.0 0.05 | 0.0 0.1 | 0.5 0.5 | 1-3, 8-10, 22-24 1, 8, 22 |
| 4 | rIL-21 Rituxan | IV IV | 0.5 10 | 1.0 4.0 | 0.5 2.5 | 1-3,8-10 1, 8 |
| 5 | rIL-21 Control Article Rituxan^{c} | IV IV IV | 1.5 0 0.05 | 3.0 0.0 0.1 | 0.5 0.5 0.5 | 1, 8, 22 2, 3, 9, 10, 23, 24 1, 8, 22 |
| 6 | rIL-21 Rituxan^{c} | IV IV | 0.5 0.05 | 1.0 0.1 | 0.5 0.5 | 1-3, 8-10, 22-24 1, 8, 22 |
| 7 | rIL-21 Rituxan^{c} | SC IV | 0.5 0.05 | 3.0 0.1 | 0.17 0.5 | 1-3, 8-10, 22-24 1,8,22 |

| | | | | | | |
|---|---|---|---|---|---|---|
| ^{a} In groups with rIL-21 and Rituxan co-administrations, Rituxan was administered before rIL-21. ^{b} Individual animal dosing volume (ml) was calculated based on the most recent body weight. Dose volumes were rounded up to the next readable syringe increment. ^{c} Rituxan dose was followed with saline flush of 3.0 ml/kg. | | | | | | |

Peripheral blood cell subsets were analyzed using flow cytometry. Approximately 1.3 ml of collected blood was placed in a tube treated with EDTA-2K, once during acclimation of Day -8, prior to dosing and six hours post-dose on Day 1, 8, and 22. Pre-dose samples were taken on Days 3, 10, and 24. Samples were taken once on Days 7, 14, 17 and 42. Approximately 0.5 ml of the sample was aliquoted for hematology analysis and the remaining sample held at room temperature until processing flow cytometry analysis.

Approximately 2.0 ml of whole blood was collected into tubes containing lithium heparin during acclimation on Day -8 and -4. It was also collected prior to dosing on Days 3, 10, 22 and 24, and once on Days 7 and 14. Samples were stored at room temperature until processing for flow cytometry analyses and ADCC activity assays.

**Table 4**

| **Antigen Markers** | **Cell Type Identified** |
|---|---|
| CD45/CD20/CD21/CD40 | CD45⁺/CD20⁻/CD21⁺ B cell |
| | CD45⁺/CD20⁺/CD21⁻ B cell |
| | CD45⁺/CD20⁺/CD21⁺ B cell |
| | CD45⁺/CD20^{high} B cell |
| | CD45⁺/CD20^{hlgh}/CD21⁻/CD40⁻ B cell |
| | CD45⁺/CD20^{high}/CD21-/CD40⁺ B cell |
| | CD45⁺/CD20^{high}/CD21⁺/CD40⁺ B cell |
| | CD45⁺/CD20^{low} B cell |
| | CD45⁺/CD20^{low}/CD21⁻/CD40⁺ B cell |
| | CD45⁺/CD20^{low}/CD21⁺/CD40⁺ B cell |
| CD45/CD14/CD16/CD64 | CD45⁺/CD14⁻/CD16⁺ Natural killer cell |
| | CD45⁺/CD14⁺/CD16⁻ Monocyte |
| | CD45⁺/CD14⁺/CD16⁺ Monocyte |
| | CD45⁺/CD14⁺/CD64⁻ Monocyte |
| | CD45⁺/CD14⁺/CD64⁺ Monocyte |
| | CD45⁺/CD64⁻ Granulocyte |
| | CD45⁺/CD64⁺ Granulocyte |
| CD45/CD3/CD8/CD11b + 11c | CD45⁺/CD3⁺/CDB⁻ T helper cell |
| | CD45⁺/CD3⁺/CD8⁺ T cytotoxic cell |
| | CD45⁺/CD3⁻/CD8⁺ NK cell |
| | CD45⁺/CD14⁻/CD16⁺ NK cell |
| | All CD45⁺/CD11b + 11c^{dim} cell |
| | All CD45⁺/CD11b + 11c^{bright} cell |
| | CD45⁺/CD3⁻/CD11b + 11c^{dim} cell |
| | CD45⁺/CD3⁻/CD11b + 11c^{bright} cell |
| | CD45⁺/CD3⁻/CD11b + 11c^{neg} cell |

The IL-21 treatment had marked effects on the phenotype and numbers of circulating leukocytes. Shortly after treatment with IL-21, all lymphocyte populations were decreased. B cells recovered more quickly than T cells and NK cells. T cells were restored to baseline levels by 4-6 days after dosing, and T helper cells were slightly elevated 4-6 days following the second cycle of IL-21 treatment. NK cells were decreased in all groups, with only partial recovery between dosing cycles. The number of circulating monocytes increased following IL-21 treatment, and both monocytes and granulocytes had increased Fc receptor expression.

### A. Lymphocyte effects

Sub-clinical doses of rituximab reduced the number of circulating B cells to a nadir 70% below baseline within 6 hr of dosing. Treatment with rIL-21 alone initially reduced circulating B cells, T cells and NK cells, followed by a sustained increase and resolution prior to the next dosing cycle. Based on the rapid reversal of lymphopenia and previous observations of lymphoid follicle depletion with rIL-21, this effect was interpreted as transient margination of activated lymphocytes combined with increased recirculation from lymphoid tissues to blood. The increase in peripheral B cells was largely mitigated in animals treated with both rIL-21 and rituximab, and a consistently lower B cell nadir was observed, relative to groups treated with rituximab or rIL-21 alone. Changes in other lymphocyte subsets induced by rIL-21 were not altered by rituximab treatment.

**Table 5 CD20low B cells in peripheral blood (counts per ul)**

| Treatment | Day -8 | Day1 | Day 1.25 | Day 3 | Day 7 | Day 8 | Day 8.25 | Day 10 | Day 14 | Day 17 | Day 22 | Day 22.25 | Day 24 | Day 29 | Day 32 | Day 37 | Day 42 |
|---|---|---|---|---|---|---|---|---|---|---|---|---|---|---|---|---|---|
| Control | 918 | 605 | 1183 | 855 | 620 | 791 | 1285 | 965 | 805 | 863 | 1145 | 1240 | 838 | 777 | 815 | 882 | 739 |
| Control | 1545 | 980 | 1464 | 1416 | 1035 | 934 | 1999 | 1233 | 1050 | 1099 | 1027 | 1507 | 1235 | 1151 | 1202 | 947 | 909 |
| Control | 1044 | 876 | 1648 | 1182 | 1179 | 807 | 1827 | 1145 | 1275 | 1046 | 888 | 1732 | 1161 | 897 | 1053 | 1205 | 944 |
| IL-21 0.5 mg/kg | 677 | 650 | 461 | 598 | 1043 | 7311 | 292 | 1241 | 871 | 835 | 507 | 231 | 307 | 717 | 553 | 380 | 426 |
| IL-21 0.5 mg/kg | 3159 | 2254 | 1684 | 3418 | 9268 | 4208 | 2136 | 9414 | 8355 | 5929 | 3887 | 1235 | 4702 | 7469 | 3657 | 3214 | 4338 |
| IL-21 0.5 mg/kg | 1310 | 1107 | 858 | 1486 | 4748 | 2378 | 1176 | 5343 | 6210 | 4139 | 1970 | 769 | 1562 | 4227 | 3122 | 2298 | 2292 |
| Ritux 0.05 mg/kg | 549 | 599 | 369 | 449 | 441 | 325 | 227 | 305 | 331 | 397 | 390 | 669 | 394 | 634 | 381 | 534 | 419 |
| Ritux 0.05 mg/kg | 2150 | 1892 | 657 | 1459 | 1993 | 1872 | 648 | 1580 | 1830 | 1810 | 1965 | 1572 | 1757 | 2133 | 1949 | 1820 | 1446 |
| Ritux 0.05 mg/kg | 1430 | 1103 | 879 | 936 | 920 | 893 | 407 | 617 | 648 | 861 | 1015 | 1127 | 834 | 1160 | 1103 | 818 | 657 |
| IL-21 0.5 mg/kg + Ritux 10 mg/kg | 687 | 407 | 36 | 9 | 4 | 3 | 2 | 0 | 2 | 2 | 2 | 2 | 1 | 7 | 15 | 2 | 5 |
| IL-21 0.5 mg/kg + Ritux 10 mg/kg | 1214 | 1116 | 69 | 5 | 1 | 1 | 1 | 2 | 1 | 6 | 12 | 3 | 3 | 9 | 59 | 102 | 182 |
| IL-21 0.5 mg/kg + Ritux 10 mg/kg | 2072 | 1846 | 157 | 17 | 2 | 4 | 3 | 0 | 0 | 3 | 12 | 12 | 12 | 110 | 192 | 564 | 707 |
| IL-21 0.5 mg/kg + Ritux 0.05 mg/kg | 597 | 603 | 283 | 356 | 1578 | 1386 | 14 | 511 | 646 | 488 | 512 | 169 | 222 | 699 | 564 | 546 | 540 |
| IL-21 0.5 mg/kg + Ritux 0.05 mg/kg | 897 | 782 | 331 | 1375 | 1622 | 1247 | 49 | 1393 | 944 | 661 | 540 | 148 | 726 | 468 | 354 | 798 | 706 |
| I L-21 0.5 mg/kg + Ritux 0.05 mg/kg | | 729 | 134 | 565 | 1560 | 1779 | 9 | 533 | 780 | 745 | 669 | 148 | 812 | 1119 | 845 | 902 | 928 |

**Table 6 CD20high B cells in peripheral blood (counts per ul)**

| Treatment | Day -8 | Day 1 | Day 1.25 | Day 3 | Day 7 | Day 8 | Day 8.25 | Day 10 | Day 14 | Day 17 | Day 22 | Day 22.25 | Day 24 | Day 29 | Day 32 | Day 37 | Day 42 |
|---|---|---|---|---|---|---|---|---|---|---|---|---|---|---|---|---|---|
| Control | 509 | 408 | 493 | 447 | 491 | 607 | 440 | 425 | 386 | 393 | 594 | 423 | 430 | 387 | 496 | 523 | 392 |
| Control | 2243 | 1808 | 1705 | 1764 | 1938 | 2594 | 1624 | 1482 | 1510 | 1529 | 1662 | 1578 | 1461 | 1542 | 1645 | 1626 | 1522 |
| Control | 1414 | 1245 | 1384 | 1190 | 1438 | 1248 | 1225 | 1173 | 1277 | 1183 | 1134 | 1418 | 1238 | 1080 | 1334 | 1147 | 952 |
| IL-21 0.5 mg/kg | 949 | 701 | 576 | 452 | 581 | 584 | 242 | 396 | 391 | 518 | 412 | 209 | 226 | 470 | 592 | 354 | 442 |
| IL-21 0.5 mg/kg | 1945 | 1784 | 932 | 488 | 1790 | 1014 | 773 | 1582 | 2542 | 2886 | 2241 | 819 | 1468 | 1897 | 1956 | 996 | 2104 |
| IL-21 0.5 mg/kg | 448 | 418 | 262 | 164 | 681 | 399 | 592 | 779 | 1370 | 909 | 562 | 181 | 499 | 701 | 750 | 338 | 386 |
| Ritux 0.05 mg/kg | 541 | 500 | 0 | 4 | 57 | 57 | 0 | 3 | 67 | 78 | 160 | 224 | 147 | 97 | 304 | 299 | 152 |
| Ritux 0.05 mg/kg | 810 | 721 | 2 | 11 | 89 | 101 | 8 | 24 | 95 | 44 | 49 | 25 | 145 | 9 | 96 | 31 | 49 |
| Ritux 0.05 mg/kg | 2144 | 1579 | 0 | 18 | 170 | 141 | 1 | 16 | 79 | 68 | 88 | 380 | 265 | 32 | 167 | 227 | 240 |
| IL-21 0.5 mg/kg + Ritux 10 mg/kg | 618 | 518 | 0 | 0 | 0 | 0 | 0 | 0 | 0 | 0 | 0 | 0 | 0 | 0 | 0 | 0 | 0 |
| IL-21 0.5 mg/kg + Ritux 10 mg/kg | 954 | 882 | 0 | 0 | 0 | 0 | 0 | 0 | 0 | 0 | 0 | 0 | 0 | 0 | 13 | 4 | 6 |
| IL-21 0.5 mg/kg + Ritux 10 mg/kg | 1538 | 1333 | 0 | 0 | 0 | 0 | 0 | 0 | 0 | 0 | 0 | 1 | 1 | 0 | 18 | 16 | 64 |
| IL-21 0.5 mg/kg + Ritux 0.05 mg/kg | 1295 | 1240 | 0 | 10 | 111 | 339 | 0 | 7 | 189 | 6 | 426 | 132 | 64 | 6 | 86 | 148 | 475 |
| IL-21 0.5 mg/kg + Ritux 0.05 mg/kg | 778 | 764 | 9 | 39 | 55 | 71 | 0 | 6 | 100 | 62 | 161 | 23 | 97 | 1 | 20 | 34 | 70 |
| IL-21 0.5 mg/kg+ Ritux 0.05 mg/kg | | 163 | 0 | 7 | 24 | 99 | 0 | 5 | 63 | 102 | 161 | 46 | 18 | 1 | 5 | 7 | 18 |

**Table 7 Cytotoxic T cells (CTLs) in peripheral blood (counts per ul**

| Treatment | Day -8 | Day 1 | Day 1.25 | Day 3 | Day 7 | Day 8 | Day 8.25 | Day 10 | Day 14 | Day 17 | Day 22 | Day 22.25 | Day 24 | Day 29 | Day 32 | Day 37 | Day 42 |
|---|---|---|---|---|---|---|---|---|---|---|---|---|---|---|---|---|---|
| Control | 2760 | 2206 | 2498 | 2867 | 2629 | 2502 | 2433 | 2688 | 2301 | 2926 | 3523 | 2705 | 2388 | 2064 | 2126 | 2662 | 2005 |
| Control | 3335 | 1891 | 1522 | 2212 | 2222 | 2051 | 2001 | 2099 | 2023 | 2438 | 1930 | 1983 | 2059 | 2141 | 2299 | 1977 | 2189 |
| Control | 2462 | 2002 | 1933 | 2496 | 2359 | 1531 | 1976 | 2133 | 2352 | 2302 | 1627 | 2411 | 2001 | 1623 | 2054 | 2087 | 1681 |
| IL-21 0.5 mg/kg | 1591 | 1171 | 650 | 996 | 1498 | 1377 | 668 | 568 | 1815 | 2928 | 978 | 527 | 591 | 2159 | 1914 | 1137 | 1156 |
| IL-21 0.5 mg/kg | 3916 | 3211 | 1307 | 830 | 3642 | 1782 | 1177 | 1331 | 5046 | 6180 | 2705 | 1629 | 1020 | 4204 | 2785 | 2648 | 1674 |
| IL-21 0.5 mg/kg | 3508 | 2978 | 1135 | 1331 | 2481 | 1516 | 743 | 1260 | 2728 | 3846 | 2702 | 1016 | 776 | 3604 | 3575 | 2658 | 2755 |
| Ritux 0.05 mg/kg | 1201 | 1353 | 1093 | 975 | 1079 | 580 | 963 | 713 | 795 | 1206 | 788 | 1037 | 512 | 683 | 503 | 996 | 644 |
| Ritux 0.05 mg/kg | 4245 | 3338 | 1069 | 3741 | 3394 | 2492 | 1084 | 2975 | 3166 | 3053 | 2830 | 1405 | 2294 | 2120 | 1804 | 2335 | 2078 |
| Ritux 0.05 mg/kg | 4417 | 2961 | 3737 | 3709 | 3412 | 3115 | 2790 | 3668 | 2572 | 3184 | 2778 | 3721 | 2582 | 2140 | 2316 | 2474 | 2335 |
| IL-21 0.5 mg/kg + Ritux 10 mg/kg | 2990 | 2613 | 840 | 381 | 1502 | 1005 | 548 | 1093 | 2478 | 4025 | 4224 | 4392 | 2690 | 4328 | 2943 | 3346 | 2627 |
| IL-21 0.5 mg/kg + Ritux 10 mg/kg | 3689 | 2888 | 1069 | 855 | 2323 | 1316 | 499 | 1480 | 3742 | 4043 | 33721 | 3123 | 1705 | 3156 | 2541 | 4294 | 2321 |
| IL-21 0.5 mg/kg + Ritux 10 mg/kg | 2636 | 3410 | 654 | 790 | 3573 | 2615 | 863 | 1671 | 3470 | 3947 | 3325 | 2812 | 1737 | 3297 | 2092 | 2588 | 1813 |
| IL-21 0.5 mg/kg + Ritux 0.05 mg/kg | 1035 | 1233 | 453 | 435 | 970 | 1531 | 211 | 763 | 1591 | 1576 | 1457 | 297 | 230 | 1087 | 1047 | 909 | 528 |
| IL-21 0.5 mg/kg + Ritux 0.05 mg/kg | 1555 | 1413 | 653 | 880 | 1150 | 1357 | 323 | 640 | 1273 | 716 | 892 | 304 | 183 | 571 | 452 | 800 | 570 |
| IL-21 0.5 mg/kg + Ritux 0.05 mg/kg | | 2244 | 781 | 672 | 1317 | 2156 | 362 | 679 | 1644 | 18831 | 2122 | 636 | 455 | 2021 | 1660 | 1652 | 1142 |

**Table 8 T-helper cells in peripheral blood (counts per ul)**

| Treatment | Day -8 | Day 1 | Day 1.25 | Day 3 | Day 7 | Day 8 | Day 8.25 | Day 10 | Day 14 | Day 17 | Day 22 | Day 22.25 | Day 24 | Day 29 | Day 32 | Day 37 | Day 42 |
|---|---|---|---|---|---|---|---|---|---|---|---|---|---|---|---|---|---|
| Control | 2163 | 2052 | 2156 | 2044 | 2178 | 2543 | 2676 | 2499 | 2404 | 2504 | 3328 | 2392 | 2485 | 2254 | 2342 | 2692 | 1949 |
| Control | 4533 | 3187 | 2327 | 3400 | 3515 | 3659 | 3062 | 3303 | 3409 | 3845 | 3183 | 2768 | 3460 | 3493 | 3772 | 3244 | 3483 |
| Control | 5205 | 4603 | 4403 | 4871 | 4798 | 4195 | 4516 | 5174 | 4861 | 5336 | 4055 | 4887 | 4979 | 4189 | 5164 | 4672 | 3833 |
| IL-21 0.5 mg/kg | 2476 | 2489 | 1033 | 1734 | 2672 | 2400 | 904 | 1069 | 2806 | 3834 | 2153 | 624 | 1455 | 3475 | 3179 | 2429 | 2336 |
| IL-21 0.5 mg/kg | 5519 | 4727 | 2445 | 1475 | 5465 | 3086 | 1791 | 3498 | 7522 | 8228 | 4390 | 2247 | 2904 | 5849 | 4633 | 4771 | 5752 |
| IL-21 0.5 mg/kg | 4181 | 3811 | 1505 | 2134 | 3428 | 2102 | 1173 | 2152 | 3814 | 4795 | 4109 | 1561 | 1942 | 4579 | 4988 | 3906 | 3619 |
| Ritux 0.05 mg/kg | 1381 | 1439 | 1391 | 1228 | 1394 | 905 | 1439 | 1145 | 1219 | 1665 | 1345 | 1525 | 1048 | 1721 | 1471 | 1814 | 1121 |
| Ritux 0.05 mg/kg | 5265 | 4916 | 2477 | 4685 | 4994 | 5234 | 2956 | 5037 | 4991 | 5209 | 5112 | 2475 | 4826 | 5337 | 4818 | 5152 | 4455 |
| Ritux 0.05 mg/kg | 5378 | 4438 | 4687 | 5168 | 5362 | 5097 | 4099 | 5604 | 4144 | 4601 | 4638 | 4835 | 4405 | 5098 | 5032 | 4772 | 4062 |
| IL-21 0.5 mg/kg + Ritux 10 mg/kg | 3160 | 2905 | 961 | 768 | 2057 | 1416 | 750 | 1790 | 3418 | 4019 | 4376 | 3457 | 3257 | 4825 | 3661 | 3669 | 2601 |
| IL-21 0.5 mg/kg + Ritux 10 mg/kg | 3837 | 3166 | 1324 | 1288 | 2817 | 1725 | 626 | 1886 | 4039 | 3590 | 3038 | 2784 | 2185 | 3282 | 2665 | 3629 | 2742 |
| IL-21 0.5 mg/kg + Ritux 10 mg/kg | 4053 | 4970 | 1414 | 1689 | 4844 | 3214 | 1012 | 2709 | 5052 | 5337 | 4600 | 3659 | 2848 | 4858 | 3513 | 4056 | 3090 |
| IL-21 0.5 mg/kg + Ritux 0.05 mg/kg | 1842 | 2234 | 841 | 1159 | 2017 | 2764 | 536 | 1815 | 2945 | 2932 | 3005 | 698 | 898 | 2577 | 2482 | 2330 | 1650 |
| IL-21 0.5 mg/kg + Ritux 0.05 mg/kg | 1953 | 1946 | 770 | 1171 | 1702 | 2360 | 430 | 894 | 2003 | 1904 | 2261 | 428 | 376 | 2806 | 2714 | 2298 | 1871 |
| I L-21 0.5 mg/kg + Ritux 0.05 mg/kg | | 1647 | 329 | 779 | 1390 | 2683 | 550 | 780 | 1536 | 1799 | 2485 | 605 | 783 | 2127 | 1944 | 1720 | 1504 |

### B. ADCC effects

MNC preparations were made with Ficoll density gradients. MNC preparations from all treated animals were characterized for immunophenotype *and ex vivo* ADCC activity during the course of this study. The target cells were loaded with Calcein-AM prior to assay, with specific release of the intracellular stain during a 3 h. incubation as the assay endpoint.

Treatment of cynomolgus monkeys with rIL-21 resulted in changes in the NK cell counts in peripheral blood and the percentage of NK cells in MNC preparations. Initially, NK cells were decreased following treatment, with a trend toward baseline values between dosing cycles.

MNCs from cynomolgus monkeys treated with rIL-21, or rituximab in combination with rIL-21, showed increased *ex vivo* ADCC activity, compared with MNCs from vehicle control and rituximab-only treated animals. Lytic activity was low on Day 3, correlating with very few NK cells in the MNC preparation. On Days 7 and 10, ADCC was increased over baseline in rIL-21 treated animals and similar trends were seen in animals treated with rIL-21 plus rituximab. Lytic activity per NK cell was maintained on Day 14 despite low numbers of NK cells in the MNC preparations

**Table 9 NK cell number in peripheral blood (counts/ul)**

| Treatment | Day 8 | Day 1 | Day 3 | Day 7 | Day 8 | Day 8.25 | Day 10 | Day 14 | Day 17 | Day 22 | Day 22.25 | Day 24 | Day 29 | Day 32 | Day 37 | Day 42 |
|---|---|---|---|---|---|---|---|---|---|---|---|---|---|---|---|---|
| Control | 210 | 211 | 224 | 224 | 326 | 223 | 202 | 147 | 204 | 368 | 196 | 181 | 126 | 190 | 149 | 180 |
| Control | 209 | 160 | 232 | 177 | 221 | 285 | 147 | 187 | 135 | 121 | 181 | 127 | 60 | 95 | 128 | 200 |
| Control | 339 | 470 | 565 | 525 | 388 | 443 | 462 | 533 | 560 | 335 | 381 | 496 | 263 | 476 | 531 | 290 |
| IL-21 0.5 mg/kg | 394 | 367 | 305 | 859 | 573 | 145 | 287 | 650 | 1077 | 355 | 84 | 182 | 365 | 332 | 249 | 210 |
| IL-21 0.5 mg/kg | 452 | 546 | 196 | 594 | 546 | 141 | 502 | 845 | 805 | 284 | 143 | 176 | 221 | 277 | 275 | 563 |
| IL-21 0.5 mg/kg | 2135 | 183 9 | | 122 0 | 927 | 198 | 550 | 1057 | 1249 | 1036 | 209 | 239 | 1104 | 852 | 1109 | 956 |
| I L-21 0.5 mg/kg + Ritux 0.05 mg/kg | 268 | 211 | 89 | 179 | 271 | 26 | 242 | 335 | 156 | 173 | 62 | 53 | 143 | 105 | 141 | 81 |
| IL-21 0.5 mg/kg + Ritux 0.05 mg/kg | 231 | 164 | 211 | 133 | 211 | 23 | 241 | 88 | 55 | 61 | 18 | 146 | 72 | 60 | 88 | 152 |
| IL-21 0.5 mg/kg + Ritux 0.05 mg/kg | | 286 | 149 | 528 | 506 | 38 | 277 | 281 | 221 | 276 | 80 | 225 | 268 | 256 | 255 | 174 |
| IL-21 0.5 mg/kg + Ritux 10 mg/kg | 579 | 399 | 153 | 365 | 218 | 81 | 408 | 464 | 469 | 224 | 161 | 139 | 330 | 251 | 405 | 119 |
| IL-21 0.5 mg/kg + Ritux 10 mg/kg | 726 | 476 | 236 | 555 | 240 | 56 | 355 | 448 | 673 | 386 | 177 | 178 | 256 | 178 | 390 | 212 |
| IL-21 0.5 mg/kg + Ritux 10 mg/kg | 337 | 442 | 310 | 310 | 154 | 67 | 525 | 482 | 1570 | 220 | 170 | 53 | 221 | 118 | 166 | 167 |
| Ritux 0.05 mg/kg | 235 | 240 | 191 | 179 | 183 | 67 | 137 | 176 | 235 | 167 | 129 | 129 | 108 | 97 | 191 | 109 |
| Ritux 0.05 mg/kg | 1265 | 794 | 948 | 824 | 712 | 125 | 795 | 836 | 638 | 745 | 398 | 623 | 681 | 542 | 741 | 615 |
| Ritux 0.05 mg/kg | 860 | 300 | 395 | 382 | 3661 | 102 | 361 | 283 | 294 | 290 | 225 | 268 | 223 | 175 | 210 | 276 |

**Table 10 NK cells as percentage of total MNC preparation**

| Day_Num | Day -8 | Day 3 | Day 7 | Day 10 | Day 14 | Day 22 | Day 24 |
|---|---|---|---|---|---|---|---|
| Control | 3.6 | 3.2 | 3.5 | 2.9 | 3.05 | 4.5 | 6.6 |
| Control | 3.73 | 4.05 | 4.8 | 4.9 | 4.9 | 4.65 | 6.05 |
| Control | 7.87 | 7.45 | 8.5 | 7.25 | 7.15 | 7.65 | 11.45 |
| IL-21 0.5 mg/kg 3d | 8.03 | 1.9 | 4.6 | 1.1 | 4.95 | 8.55 | 2.1 |
| IL-21 0.5 mg/kg 3d | 8.4 | 0.35 | 3.65 | 0.55 | 4.65 | 8.15 | 0.65 |
| I L-21 0.5 mg/kg 3d | 17.97 | 2.95 | 5.4 | 1.45 | 4.3 | 11.5 | 2.9 |
| Rituxan 0.05 mg/kg | 7.77 | 6.05 | 7 | 5 | 8.75 | 8.1 | 8.2 |
| Rituxan 0.05 mg/kg | 7.63 | 6.75 | 8.45 | 6.35 | 9.3 | 8.35 | 9.55 |
| Rituxan 0.05 mg/kg | 6.17 | 3.95 | 4.4 | 2.45 | 3.7 | 4.25 | 6.25 |
| Rituxan 10 mg/kg + IL-21 0.5 mg/kg | 10.03 | 0.95 | 2.05 | 0.7 | 1.8 | 3.05 | 5.2 |
| Rituxan 10 mg/kg + IL-21 0.5 mg/kg | 9.6 | 0.35 | 2.85 | 0.55 | 1.75 | 5 | 6.3 |
| Rituxan 10 mg/kg + IL-21 0.5 mg/kg | 2.7 | 0.3 | 1.05 | 0.55 | 1 | 2.25 | 0.9 |
| Rituxan 0.05 mg/kg + IL210.5 mg/kg | 8.63 | 0.45 | 1.5 | 1.15 | 4.15 | 4.8 | 0.45 |
| Rituxan 0.05 mg/kg + IL21 0.5 mg/kg | 6.17 | 0.5 | 0.95 | 0.3 | 1.8 | 2.75 | 0.65 |
| Rituxan 0.05 mg/kg + IL210.5 mg/kg | 5.7 | 0.65 | 1.75 | 0.55 | 2.25 | 3.85 | 1 |

**Table 11 ADCC in cynomolgus monkeys treated with rIL-21. From whole MNC prep percent lysis at E:T ratio of 25.**

| Treatment | Cyno_ID | Target | Effector | Day -4 | Day 3 | Day 7 | Day 10 | Day 14 | Day 22 | Day 24 |
|---|---|---|---|---|---|---|---|---|---|---|
| Control | Cyno 1 | BT474-2 | MNC | 8.53 | 3.87 | 12.24 | 10.49 | 12.16 | 13.74 | 15.7 |
| Control | Cyno 2 | BT474-2 | MNC | 17.07 | 12.93 | 18.02 | 16.42 | 11.17 | 19.27 | 13.41 |
| Control | Cyno 3 | BT474-2 | MNC | 18.57 | 6.85 | 9.38 | 6.71 | 13.67 | 16.36 | 19.63 |
| rIL-21 0.5 mg/kg | Cyno 4 | BT474-2 | MNC | 23.13 | 9.45 | 46.34 | 25.28 | 29.78 | 24.14 | 9.72 |
| r1L-21 0.5 mg/kg | Cyno 5 | BT474-2 | MNC | 30.73 | 2.2 | 48.52 | 13.76 | 26.92 | 29.34 | |
| rIL-21 0.5 mg/kg | Cyno 6 | BT474-2 | MNC | 28.72 | 21.56 | 36.07 | 21.82 | 32.01 | 34.4 | 20.91 |
| rIL-21 0.5 mg/kg + Rituxan 0.05 mg/kg | Cyno 16 | BT474-2 | MNC | 28 | 8.52 | 28.87 | 45.03 | 24.43 | 28.44 | 8.5 |
| rIL-21 0.5 mg/kg + Rituxan 0.05 mg/kg | Cyno 17 | BT474-2 | MNC | 26.9 | 1.57 | 17.7 | 13.61 | 10.23 | 14.8 | 2.21 |
| rIL-21 0.5 mg/kg + Rituxan 0.05 mg/kg | Cyno 22 | BT474-2 | MNC | 33.07 | 7.55 | 17.63 | 20.92 | 19.81 | 14.48 | 8.95 |
| rIL-21 0.5 mg/kg + Rituxan 10 mg/kg | Cyno 10 | BT474-2 | MNC | 34.37 | 7.19 | 34 | 27.55 | 20.57 | 23.22 | 18.01 |
| rIL-21 0.5 mg/kg + Rituxan 10 mg/kg | Cyno 11 | BT474-2 | MNC | 33.82 | 5.87 | 29 | 20.84 | 15.24 | 30.09 | 22.44 |
| rIL-21 0.5 mg/kg + Rituxan 10 mg/kg | Cyno 12 | BT474-2 | MNC | 13.43 | 9.42 | 19.69 | 15.35 | 8.03 | 9.31 | 7 |
| Rituxan 0.05 mg/kg | Cyno 7 | BT474-2 | MNC | 27.91 | 15.51 | 30.78 | 25.22 | 33.84 | 25.26 | 25.86 |
| Rituxan 0.05 mg/kg | Cyno 8 | BT474-2 | MNC | 27.98 | 17.37 | 23.59 | 19.79 | 16.26 | 29.7 | 19.69 |
| Rituxan 0.05 mg/kg | Cyno 9 | BT474-2 | MNC | 33.81 | 6.78 | 20.15 | 16.21 | 14.52 | 17.47 | 26.16 |

**Table 12 Percent specific lysis measured using ADCC in cynomolgus monkeys treated with rIL-21. NK-adjusted ADCC for an E:T ratio of 2.**

| Treatment | Cyno_ID | Target | Effecto r | Day -4 | Day 3 | Day 7 | Day 10 | Day 14 | Day 22 | Day 24 |
|---|---|---|---|---|---|---|---|---|---|---|
| Control | Cyno 1 | BT474-2 | NK_L | 9.43 | 4.71 | 16.24 | 18.46 | 14.41 | 14.35 | 15.83 |
| Control | Cyno 2 | BT474-2 | NK_L | 19.07 | 15.09 | 21.23 | 20.38 | 11.17 | 21.93 | 14.04 |
| Control | Cyno 3 | BT474-2 | NK_L | 18.45 | 7.12 | 8.98 | 7.51 | 13.77 | 16.47 | 18.88 |
| rIL-21 0.5 mg/kg | Cyno 4 | BT474-2 | NK_L | 23.1 | 15.98 | 52.37 | 63.06 | 31.84 | 23.77 | 10.28 |
| rIL-21 0.5 mg/kg | Cyno 5 | BT474-2 | NK_L | 30.52 | 2.99 | 57.41 | 49.07 | 29.02 | 29.32 | |
| rIL-21 0.5 mg/kg | Cyno 6 | BT474-2 | NK_L | 27.36 | 28.1 | 39.81 | 24.36 | 37.28 | 31.91 | 32.19 |
| rIL-21 0.5 mg/kg + Rituxan 0.05 mg/kg | Cyno 16 | BT474-2 | NK_L | 27.95 | 10.39 | 35.81 | 57.8 | 25.19 | 29.25 | 8.51 |
| rIL-21 0.5 mg/kg + Rituxan 0.05 mg/kg | Cyno 17 | BT474-2 | NK_L | 27.14 | 1.57 | 19.63 | 20.07 | 10.23 | 17.33 | |
| rIL-21 0.5 mg/kg + Rituxan 0.05 mg/kg | Cyno 22 | BT474-2 | NK_L | 31.2 | 7.57 | 23.16 | 21.63 | 21.63 | 14.48 | 8.95 |
| rIL-21 0.5 mg/kg + Rituxan 10 mg/kg | Cyno 10 | BT474-2 | NK_L | 33.53 | 9.87 | 46.66 | 30.62 | 26.89 | 25.51 | 19.54 |
| rIL-21 0.5 mg/kg + Rituxan 10 mg/kg | Cyno 11 | BT474-2 | NK_L | 33.5 | 6.71 | 37.37 | 27.43 | 15.89 | 31.52 | 22.95 |
| rIL-21 0.5 mg/kg + Rituxan 10 mg/kg | Cyno 12 | BT474-2 | NK_L | 13.43 | 15.24 | 37.3 | 15.56 | 9.89 | 9.31 | 7 |
| Rituxan 0.05 mg/kg | Cyno 7 | BT474-2 | NK_L | 27.7 | 16.68 | 32.32 | 27.36 | 33.62 | 25.26 | 25.82 |
| Rituxan 0.05 mg/kg | Cyno8 | BT474-2 | NK_L | 26.03 | 18.13 | 23.11 | 21.31 | 16.24 | 29.56 | 19.67 |
| Rituxan 0.05 mg/kg | Cyno 9 | BT474-2 | NK_L | 34.07 | 7.69 | 25.72 | 22.44 | 15.06 | 21.03 | 26.84 |

### C. Additional endpoints

Soluble IL-2Rα (sCD25), an immune activation marker increased rapidly upon rIL-21 dosing, and intracellular perforin, a lytic granule enzyme increased more slowly, with highest expression following the second dosing interval. FcγRI (CD64), and FcγRIII (CD16) were up-regulated in both monocytes and granulocytes.

Perforin was measured by flow cytometry in MNC preparations. For measurement of sCD25, blood was collected once during acclimation of Day -8 and once on Days 17, 29, 37, and 42. It was also collected on Days 1, 8, and 22 prior to dosing, and five minutes, 30 minutes, two hours, and six hours post-dose. On Days 3, 10, and 24, blood was collected 30 minutes post-dose.

Approximately 0.75 ml of blood was transferred to an SST clot tube. The samples were allowed to clot at room temperature for approximately 40-60 minutes. Serum was obtained by centrifugation (2000 x g for approximately 15 minutes at 2-8°C, and were stored in a freezer at -70°C. Soluble CD25 present in serum was captured using a murine monoclonal anti-sCD25 antibody, and detected with biotinylated goat polyclonal anti-sCD25 antibody. Streptavidin-HRP and the substrate TMB allowed the colorimetric quantification of sCD25 present in samples and standards.

**Table 13**

| sCD25 content in serum (ng/ml) | | | | | | | | | |
|---|---|---|---|---|---|---|---|---|---|
| Day_num | Day -8 | Day 1 | Day 3 | Day 8 | Day 10 | Day 17 | Day 22 | Day 24 | Day 29 |
| Control | 0 | 0 | 0 | 0.79 | 0.69 | 0.73 | 0 | 0 | 0.67 |
| Control | 1.01 | 1.14 | 0.91 | 1.08 | 1.12 | 1.2 | 1.14 | 1.08 | 1.04 |
| Control | 0.75 | 0.73 | 0.85 | 0.85 | 0.97 | 0.98 | 0.97 | 0.86 | 1.31 |
| IL-21 0.5 mg/kg | 0 | 0 | 2.58 | 1.41 | 10.2 | 1.43 | 0 | 6.54 | 0.97 |
| IL-21 0.5 mg/kg | 0 | 0.81 | 4.58 | 1.64 | 8.37 | 1.21 | 0.81 | 5.16 | 1.29 |
| IL-21 0.5 mg/kg | 0 | 0 | 2.49 | 1.73 | 9.29 | 1.4 | 0.8 | 5.65 | 1.43 |
| Rituxan 0.05 mg/kg | 0 | 0 | 0 | 0 | 0 | 0 | 0 | 0 | 0 |
| Rituxan 0.05 mg/kg | 0 | 0 | 0 | 0 | 0 | 0 | 0 | 0 | 0 |
| Rituxan 0.05 mg/kg | 0.75 | 0.7 | 0.67 | 0.67 | 0 | 0 | 0 | 0 | 0 |
| Rituxan 10 mg/ml + IL-21 0.5 mg/kg | 0 | 0 | 3.12 | 1.29 | 9.82 | 1.11 | 0 | 0 | 0 |
| Rituxan 10 mg/ml + IL-21 0.5 mg/kg | 0 | 0 | 3.18 | 1.36 | 13.5 | 0.7 | 0 | 0 | 0 |
| Rituxan 10 mg/ml + IL-21 0.5 mg/kg | 0 | 0 | 3.51 | 0.83 | 8.31 | 0 | 0 | 0 | 0 |
| Rituxan 0.05 mg/kg + IL21 0.5 mg/kg | 0 | 0 | 1.74 | 1.3 | 9.51 | 0 | 0 | 2.76 | 0 |
| Rituxan 0.05 mg/kg + IL21 0.5 mg/kg | 0 | 0 | 4.75 | 1.83 | 13 | 0.95 | 0 | 7.98 | 0 |
| Rituxan 0.05 mg/kg + IL21 0.5 mg/kg | 0 | 0 | 3.39 | 1.29 | 6.75 | 1.48 | 0 | 5.7 | 0 |

**Table 14**

| Percentage of CTL positive for Perforin Expression | | | | | | | | |
|---|---|---|---|---|---|---|---|---|
| Day_Num | Day -8 | Day 3 | Day 7 | Day 10 | Day 14 | Day 22 | Day 24 | Day 29 |
| Control | 0.9 | 1.6 | 0.2 | 0 | 0.4 | 0 | 0 | 0.4 |
| Control | 2.3 | 1 | 1.2 | 1.1 | 5.9 | 0.1 | 0.2 | 5.9 |
| Control | 3.8 | 1.7 | 2 | 2 | 4.2 | 0.1 | 0.7 | 3.5 |
| IL-21 0.5 mg/kg | 4.7 | 10.4 | 16.7 | 34.7 | 28.3 | 0 | 0.2 | 20.1 |
| IL-21 0.5 mg/kg | 1.3 | 17.5 | 9.8 | 33.4 | 31.8 | 0 | 4.2 | 35.6 |
| IL-21 0.5 mg/kg | 4.2 | 7.6 | 19.6 | 39.4 | 24.6 | 0.2 | 12.3 | 21.4 |
| Rituxan low | 1.7 | 0.1 | 0.1 | 1 | 0.4 | 0.1 | 0 | 1.7 |
| Rituxan low | 1.3 | 0.3 | 0.1 | 2.4 | 1 | 0 | 0.1 | 3.9 |
| Rituxan low | 2.6 | 1.3 | 0.6 | 0.4 | 7.2 | 0 | 0.1 | 5.9 |
| Rituxan high + IL-21 0.5 mg/kg | 0.3 | 0.2 | 1.2 | 16.8 | 26.8 | 0 | 0.1 | 0.9 |
| Rituxan high + IL-21 0.5 mg/kg | 2.1 | 1.8 | 5.1 | 13.8 | 25.2 | 0.1 | 0.2 | |
| Rituxan high + IL-21 0.5 mg/kg | 1.1 | 14.8 | 4.6 | 18.7 | 9.8 | 0 | 0 | 9.8 |
| Rituxan low + IL-21 0.5 mg/kg | 3.1 | 16.5 | 16.2 | 34.6 | 51.6 | 0.2 | 19 | 58 |
| Rituxan low + IL-21 0.5 mg/kg | 0.7 | 3.7 | 1.9 | 5.8 | 9 | 0.1 | 5.8 | 12.9 |
| Rituxan low + IL-21 0.5 mg/kg | 3 | 6.5 | 7 | 12.4 | 20.8 | 0.1 | 3.7 | 15 |

**Table 15**

| Percentage of NK cells positive for Perforin Expression | | | | | | | | |
|---|---|---|---|---|---|---|---|---|
| Day_Num | Day -8 | Day 3 | Day 7 | Day 10 | Day 14 | Day 22 | Day 24 | Day 29 |
| Control | 0.6 | 23.2 | 0.9 | 1.8 | 2.4 | 1.7 | 0.5 | 2.3 |
| Control | 3.5 | 4.5 | 3.1 | 3.3 | 22.2 | 3.1 | 0.6 | 13 |
| Control | 17 | 5.8 | 3.5 | 6.3 | 19.1 | 1 | 4.6 | 29.8 |
| IL-21 0.5 mg/kg 3d | 1.6 | 6.4 | 17.3 | 19.2 | 56.4 | 1.2 | 1.4 | 23.6 |
| IL-21 0.5 mg/kg 3d | 0.9 | 14.5 | 4.3 | 11.1 | 43.6 | 0.8 | 2.5 | 58.9 |
| IL-21 0.5 mg/kg 3d | 6.5 | 5.4 | 23.9 | 49.4 | 43.4 | 0.8 | 10.5 | 45.5 |
| Rituxan low | 2.1 | 0.3 | 0.9 | 1.9 | 1.4 | 1.3 | 0.4 | 5.8 |
| Rituxan low | 1.8 | 0.9 | 0.4 | 7 | 2.4 | 1.5 | 0.2 | 10.3 |
| Rituxan low | 2.6 | 1.4 | 1.6 | 3.2 | 13.2 | 4.7 | 0.3 | 5.7 |
| Rituxan high + IL-21 0.5 mg/kg | 0.5 | 6.8 | 4.5 | 16.1 | 49.6 | 2.1 | 0.6 | 2.5 |
| Rituxan high + IL-21 0.5 mg/kg | 3.3 | 18.1 | 4.1 | 4.4 | 51.1 | 1.7 | 1.5 | |
| Rituxan high + IL-21 0.5 mg/kg | 0.8 | 25.9 | 3.2 | 3 | 22.3 | 7.7 | 6.7 | 32.6 |
| Rituxan low + IL21 0.5 mg/kg | 2.1 | 15 | 7.1 | 14.5 | 73.7 | 2.9 | 9.6 | 66 |
| Rituxan low + IL21 0.5 mg/kg | 1.1 | 19.8 | 2.7 | 8.9 | 8.2 | 4.7 | 9.6 | 10.9 |
| Rituxan low + IL21 0.5 mg/kg | 6.2 | 20.3 | 7.3 | 13.5 | 49 | 8.7 | 9.9 | 22.7 |

**Table 16 Granulocytes - Percentage Change from Baseline in CD64 MFI**

| | | | | | | | | | | | | | | |
|---|---|---|---|---|---|---|---|---|---|---|---|---|---|---|
| Day | Day 3 | Day 7 | Day 8 | Day 8.25 | Day 10 | Day 14 | Day 17 | Day 22 | Day 22.25 | Day 24 | Day 29 | Day 32 | Day 37 | Day 42 |
| Control | -14.0 | -3.5 | -12.9 | -11.8 | -24.2 | -2.3 | -8.6 | 5.3 | -1.0 | -8.5 | -2.9 | 0.3 | -19.6 | -25.8 |
| Control | -1.2 | 5.6 | -4.3 | -9.0 | -2.1 | 6.4- | 3.2 | 18.2 | 11.7 | 3.3 | 17.8 | 6.6 | 4.6 | -1.2 |
| Control | -17.0 | 22.2 | -12.3 | -10.8 | -5.6 | -2.4 | 11.9 | 41.0 | 20.9 | 24.0 | 27.0 | -15.9 | 10.2 | -18.3 |
| IL-21 0.5 mg/kg | -16.3 | -2.4 | -0.7 | -11.0 | 0.0 | 25.9 | 19.6 | 9.3 | 9.5 | -13.3 | 2.2 | 0.4 | -14.2 | -35.6 |
| IL-21 0.5 mg/kg | 65.0 | 143.1 | 81.4 | 110.2 | 151.8 | 160.2 | 123.7 | 97.7 | 88.0 | 77.6 | 88.9 | 1.9 | 16.1 | -10.9 |
| IL-21 0.5 mg/kg | | 68.3 | 34.2 | 39.8 | 76.2 | 74.8 | 71.1 | 74.1 | 83.1 | 64.2 | 41.7 | -9.4 | 12.9 | -15.2 |
| Rituxan 0.05 mg/ml | -0.3 | 0.7 | 10.3 | 9.8 | 7.9 | 2.2 | 13.5 | 10.7 | 15.5 | 0.1 | 21.1 | 8.8 | 4.7 | -2.0 |
| Rituxan 0.05 mg/ml | -11.8 | 15.6 | -10.0 | -18.0 | -15.3 | -4.2 | 6.1 | 18.3 | 17.7 | 0.0 | 22.0 | -10.7 | -1.9 | -31.5 |
| Rituxan 0.05 mg/ml | 0.0 | 1.81 | -8.7 | 0.4 | -9.5 | -6.4 | 20.8 | 21.3 | 22.1 | 21.8 | 31.9 | 4.1 | 0.2 | 7.7 |
| Rituxan 10 mg/ml + IL-21 0.5 mg/kg | 2.9 | 41.1 | 21.5 | 9.5 | 35.3 | 83.1 | 28.0 | 15.9 | 21.9 | 26.2 | -2.4 | -29.8 | -14.6 | -10.0 |
| Rituxan 10 mg/ml + IL-21 0.5 mg/kg | 5.7 | 48.3 | 16.2 | 10.9 | 41.7 | 73.2 | 60.6 | 45.5 | 42.2 | 24.8 | 22.1 | -1.3 | 2.0 | -5.7 |
| Rituxan 10 mg/ml+ IL-21 0.5 mg/kg | -16.5 | 76.5 | 69.0 | 77.9 | 113.1 | 313.4 | 144.8 | 99.1 | 137.1 | 85.8 | 15.3 | -12.9 | -27.4 | -11.6 |
| Rituxan 0.05 mg/ml + IL21 0.5 mg/kg | -1.5 | 138.8 | 130.6 | 101.1 | 122.2 | 256.0 | 176.2 | 47.8 | 35.7 | 42.2 | 162.5 | 94.7 | 16.3 | 4.0 |
| Rituxan 0.05 mg/ml + IL21 0.5 mg/kg | 26.1 | 36.2 | 26.0 | 21.5 | 63.6 | 92.9 | 68.9 | 41.9 | 57.9 | 46.3 | 12.0 | -3.0 | -2.4 | -11.6 |
| Rituxan 0.05 mg/ml + IL21 0.5 mg/kg | 40.7 | 30.3 | 56.3 | 97.1 | 154.5 | 251.6 | 129.8 | 35.7 | 79.1 | 43.9 | 92.0 | 62.2 | 57.9 | 19.2 |

**Table 17 Monocytes - Percentage Change from Baseline in CD64 MFI**

| | | | | | | | | | | | | | | |
|---|---|---|---|---|---|---|---|---|---|---|---|---|---|---|
| Day | Day 3 | Day 7 | Day 8 | Day 8.25 | Day 10 | Day 14 | Day 17 | Day 22 | Day 22.25 | Day 24 | Day 29 | Day 32 | Day 37 | Day 42 |
| Control | -6.0 | -25.1 | -23.8 | -17.8 | -0.2 | -2.1 | 3.4 | 2.0 | -1.2 | -1.5 | 10.1 | -15.5 | -6.7 | -9.8 |
| Control | -12.5 | -18.0 | -30.5 | -6.5 | -10.6 | 0.9 | 12.8 | 6.1 | 15.3 | 12.4 | 13.3 | -35.7 | 1.2 | 8.5 |
| Control | -13.7 | -13.6 | -23.0 | -10.9 | 11.0 | 4.6 | 10.0 | 10.4 | 12.1 | 4.1 | 6.3 | -31.1 | -17.7 | 11.6 |
| IL-21 0.5 mg/kg | 74.2 | 32.7 | 12.1 | 36.1 | 127.6 | 35.4 | 10.2 | 0.3 | 23.9 | 105.2 | 2.7 | -22.1 | -1.1 | -25.9 |
| IL-21 0.5 mg/kg | 346.4 | 119.0 | 39.3 | 122.3 | 322.5 | 112.0 | 37.9 | 19.2 | 54.5 | 301.2 | 66.2 | -10.6 | 30.7 | 7.8 |
| IL-21 0.5 mg/kg | | 65.4 | 23.4 | 125.1 | 230.4 | 67.0 | 38.1 | 18.4 | 82.4 | 185.2 | 39.5 | -18.6 | -13.6 | 13.9 |
| Rituxan 0.05 mg/ml | -14.8 | -11.5 | -17.3 | -1.3 | -3.8 | -8.6 | -10.6 | -6.3 | 7.3 | -16.0 | -11.8 | -12.3 | -8.1 | -2.7 |
| Rituxan 0.05 mg/ml | -21.0 | -3.9 | -16.8 | 16.6 | -11 .1 | 7.3 | 5.6 | 3.5 | 22.6 | 13.7 | 9.9 | -12.6 | 15.9- | 14.2 |
| Rituxan 0.05 mg/ml | -6.9 | 4.4 | -21.1 | 2.5 | 8.2 | 16.1 | 17.3 | 13.8 | 10.8 | 15.6 | 24.2 | -24.1 | 25.4 | 50.6 |
| Rituxan 10 mg/ml + IL-21 0.5 mg/kg | 236.5 | 123.6 | 77.5 | 182.4 | 320.9 | 114.1 | 57.9 | 23.8 | 32.8 | 11.8 | 21.2 | 8.0 | 31.0 | 60.5 |
| Rituxan 10 mg/ml + IL-21 0.5 mg/kg | 249.5 | 123.3 | 68.1 | 173.8 | 281.8 | 62.3 | 75.7 | 19.1 | 22.6 | 16.4 | 26.0 | -2.5 | 35.2 | 5.9 |
| Rituxan 10 mg/ml + IL-21 0.5 mg/kg | 334.9 | 105.2 | 52.6 | 115.1 | 393.7 | 135.5 | 107.4 | 2.7 | 8.3 | 5.2 | 29.1 | -3.0 | 21.6 | -3.5 |
| Rituxan 0.05 mg/ml + IL21 0.5 mg/kg | 225.5 | 114.3 | 76.2 | 159.7 | 304.1 | 89.9 | 46.2 | 33.1 | 58.1 | 281.6 | 58.2 | 7.6 | 21.3 | 13.3 |
| Rituxan 0.05 mg/ml + IL21 0.5 mg/kg | 208.9 | 148.7 | 83.3 | 194.4 | 368.5 | 89.4 | 43.0 | 28.3 | 82.5 | 245.1 | 39.0 | -4.6 | 9.5 | 10.9 |
| Rituxan 0.05 mg/ml + IL21 0.5 mg/kg | 196.9 | 183.6 | 128.5 | 303.1 | 356.7 | 108.2 | 26.5 | 15.3 | 55.9 | 193.6 | 11.9 | -10.4 | 48.8 | 20.8 |

### Example 6

### IL-21 and trastuzumab-mediated killing of Her-2/neu Expressing Breast Cancer Cell Lines

### A.

Two hundred mL human blood was obtained from a donor program. 180 mL of blood was collected in acid citrate dextrose tubes and 20 mL from the same donor was collected in clot tubes (BD Biosciences). The blood in clot tubes was centrifuged at 2800 rpm for 30 minutes. The serum was harvested off the top and used in the culture media (see below). The 180 mL of blood in the ACD tubes was pooled and diluted 1:2 in phosphate buffer saline (PBS), 2% fetal bovine serum (FBS). 30 mL aliquots of blood were put into 50 mL tubes. 12 mL Ficoll-Paque PLUS (Amersham Biosciences) was layered on the bottom of each of the 50 mL tubes of blood. Tubes of blood were centrifuged at 1800 rpm for 30 minutes. The buffy coat interface was collected from each 50 mL tube and pooled. The pools were washed 2 - 3 times with a total of 100x cell volume PBS, 2%FBS. The final washed pellet was re-suspended in 2 mL PBS, 2% FBS. Cells were counted on a hemocytometer.

MNC cells purified as described above were cultured at .5x10⁶ cells/mL in SF Complete (αMEM with nucleosides, 50 µM B-mercaptoethanol, 1: 100 insulin, transferring, selenium stock (Invitrogen), 150 µg/mL additional transferrin, 5 mg/mL bovine serum albumin) with 4% autologous serum with or without the addition of 20 ng/mL human IL-21 for 4 days at 37°C. On day 4 cells were harvested, counted on the hemocytometer and washed in Hank's Buffered Salt Solution (HBSS without Ca or Mg) with 5% fetal bovine serum (FBS), now called HBSSF. Cell pellets were re-suspended in HBSSF to .5x10⁶ cells/ mL.

Breast cancer target cell lines, including BT-474 (ATCC No. HTB-20), SK-BR-3 (ATCC No. HTB-30), or MCF-7 (ATCC No. HTB-22), were labeled with 10 µM calcein in HBSSF for 1 hour at 37°C. Targets were then washed in 10 volumes of HBSSF at 1100 rpm for 8 minutes. Cell pellets were resuspended in HBSSF to 50,000 cells/mL. MNC effector cells pretreated with or without human IL-21 for 4 days as described above were centrifuged at 1100 rpm for 8 minutes and cell pellets re-suspended in HBSSF to about .5x10⁶ cells/ mL. Effectors were serially diluted 1:3 in 96-well round bottom plates in duplicates. 100 µl targets were added to each well in the presence of either 2 µg/mL human IgG, or 2.5 µg/ mL trastuzumab. 96- well plates were centrifuged at 500 rpm for 3 minutes. Plates were incubated at 37°C for 3 hours and then centrifuged at 1000 rpm for 5 minutes. 100 µl supernatant from each well was transferred to 96 - well flat-bottomed plates and read on the Wallac fluorometer (Wallac) at 485/535 with 1 second intervals.

The MCF-7 breast cancer cell line expresses Her-2/neu antigen at low levels compared to the BT-474 cell line, which expresses this antigen at high levels. In the ADCC assay described above, when MCF-7 targets are used at an effector:target (E:T) ratio of 3, untreated effectors in the presence of trastuzumab have no more cytolytic activity than untreated effectors with IgG in the assay. In the presence of trastuzumab, at an E:T of 10, IL-21 pretreated effectors have 4 fold more cytolytic activity than untreated effectors. At an E:T of 10, in the presence of IgG, the IL-21 pretreated effectors had a 0.5 fold increase in cytolytic activity over the untreated effectors. When BT-474 cells are the targets, in the presence of trastuzumab, at an E:T of 10 there is a 7-fold increase in cytolytic activity from untreated effectors over those untreated and with IgG present in the assay. At an E:T of 10, IL-21 pretreatment increases this cytolytic activity 2-fold. These results are supportive of the fact that the MCF-7 cell line is a low antigen expresser as trastuzumab alone is ineffective at enhancing effector cytolytic activity on this cell line. trastuzumab alone is effective at enhancing cytolytic activity of effectors on the BT-474 targets. IL-21 pretreatment further increases the cytolytic activity of effectors on both of these cell lines.

**Table 18 ADCC activity from human NK cells against breast cancer targets. Endpoint is percentage lysed at an E:T ratio of 3.**

| Donor | Target | Control | rIL-21 pretreatment |
|---|---|---|---|
| A74 | MCF-7 | 11.2943 | 22.71177 |
| A74 | SKBr3 | 12.12149 | 39.34667 |
| A74 | MCF-7 | 0 | 25.9 |
| B202 | HCC142 8 | 27.76922 | 54.18124 |
| B202 | HCC38 | 27.4133 | 43.12007 |
| B202 | HCC38 | 9.00991 | 40.34685 |
| B202 | MCF7 | 13.73884 | 45.35621 |
| B202 | MCF7 | 8.487321 | 39.9608 |
| B202 | MCF7 | 8.739211 | 41.61529 |
| B202 | SKBR3 | 28.50026 | 53.7579 |
| B202 | SKBR3 | 14.84613 | 26.45897 |
| C025 | HCC1428 | 18.60837 | 50.02053 |
| C025 | HCC1428 | 14.20742 | 27.40921 |
| C025 | HCC38 | 16.89381 | 32.41753 |
| C025 | HCC38 | 7.033291 | 28.39438 |
| C025 | MCF7 | 14.40028 | 45.16213 |
| C025 | MCF7 | 6.009641 | 27.19668 |
| C025 | MCF7 | 8.491816 | 23.90491 |
| C025 | MCF7 | 11.2943 | 22.71177 |
| C025 | SKBR3 | 26.61171 | 48.15062 |
| C025 | SKBR3 | 18.33489 | 34.07056 |
| C025 | SKBR3 | 12.12149 | 39.34667 |

### B.

Two hundred mL human blood was obtained from a donor program. 180 mL of blood was collected in acid citrate dextrose tubes and 20 mL from the same donor was collected in clot tubes (BD Biosciences). The blood in clot tubes was centrifuged at 2800 rpm for 30 minutes. The serum was harvested off the top and used in the culture media (see below). The 180 mL of blood in the ACD tubes was pooled and diluted 1:2 in phosphate buffer saline (PBS), 2% fetal bovine serum (FBS). 30 mL aliquots of blood were put into 50 mL tubes. 12 mL Ficoll-Paque PLUS (Amersham Biosciences) was layered on the bottom of each of the 50 mL tubes of blood. Tubes of blood were centrifuged at 1800 rpm for 30 minutes. The buffy coat interface was collected from each 50 mL tube and pooled. The pools were washed 2 - 3 times with a total of 100x cell volume PBS, 2%FBS. The final washed pellet was re-suspended in 2 mL PBS, 2% FBS. Cells were counted on a hemocytometer.

MNCs were diluted to 5 - 10 x 10⁷ cells / mL in PBS, 2% FBS. NK cells were purified using the StemCell Technologies Enrichment of Human NK Cell kit. NK cells were centrifuged at 1100 rpm for 8 minutes and re-suspended in .5 mL PBS, 2%FBS and counted on a hemocytometer.

BT-474 cells (ATCC No. HTB-20) were plated at .125 x10⁶ cells / mL in a 12 well plate in DMEM (Gibco), 10 % FBS and allowed to adhere for 3 hours. NK cells purified as above were diluted to 1 - 2 x 10⁶ cells/ mL in SF Complete (αMEM with nucleosides, 50 µM β-mercaptoethanol, 1:100 insulin, transferring, selenium stock (Invitrogen) 150µg/ mL additional transferrin, 5 mg/ mL bovine serum albumin) plus 4 % heat inactivated human AB serum. Media was aspirated off the BT-474 cells and 2 mL diluted NK cells were added to the BT-474 cells, setting up the co-culture. Nothing, 2 µg/ml trastuzumab, 20 ng/ml human IL-21 or 2 µg/mL + 20 ng/ml human IL-21 was added to the co-culture. The co-cultures were incubated overnight at 37°C. After overnight co-culture the cells were harvested and counted on a hemocytometer. Cells were washed in HBSSF (see below) and cell pellets re-suspended in 1 mL HBSSF.

20 µg/mL mouse gamma globulin (Jackson ImmunoResearch Laboratories, Inc. West Grove, PA) and 1:100 conjugated antibody was added to 100,000 - 200,000 NK cells in 100 µL HBSSF. The antibody combination included CD25FITC, CD56PE, CD16Cychrome, and CD8APC (BD Pharmingen). Isotype controls included 100,000 - 200,000 pooled cells from NK co-cultures with each conjugated antibody alone. Cells were incubated at 4 °C for 30 minutes in the dark. Cells were washed 1 time in PBS, 2%FBS and left in 200 µL PBS, 2%FBS. Paraformaldehyde was added to 0.2% to fix cells and cells were kept at 4°C until ready to do FACS analysis. FACS analysis was done on a Becton Dickinson FACS Calibur within 3-4 days of fixing. Cell quest software was used to analyze flow data. The total cell number was calculated by multiplying the number of cells per mL by the culture volume.

In all 4 donors tested, there was an increase in the CD56+/CD25+ population of cells when trastuzumab and IL-21 were in the co-culture of NK cells and the breast cell cancer line, BT-474. Specifically, when compared to the co-cultures with media alone (described above), trastuzumab alone resulted in a 2 - 20 fold increase, IL-21 alone resulted in a 2 - 4 fold increase and, when IL-21 and trastuzumab were present there was a 4 - 50 fold increase in the CD56+/CD25+ population. In all donors there was an increase in the CD56+/CD25+ population in the presence of IL-21 and trastuzumab over all other co-culture conditions.

### C.

200 mL human blood was obtained from the in house donor program. 180 mL of blood was collected in ACD tubes and 20 mL from the same donor was collected in clot tubes. The blood in clot tubes was centrifuged at 2800 rpm for 30 minutes. The serum was harvested off the top and used in the culture media (see below). The 180 mL of blood in the ACD tubes was pooled and diluted 1:2 in phosphate buffer saline (PBS), 2% fetal bovine serum (FBS). 30 mL aliquots of blood were put into 50 mL tubes. 12 mL Ficoll-Paque PLUS (Amersham Biosciences cat. No. 17-1440-03) was layered on the bottom of each of the 50 mL tubes of blood. Tubes of blood were centrifuged at 1800 rpm for 30 minutes. The buffy coat interface was collected from each 50 mL tube and pooled. The pools were washed 2-3 times with a total of 100x cell volume PBS, 2% FBS. The final washed pellet was re-suspended in 2 mL PBS, 2% FBS. Cells were counted on a hemocytometer.

MNC cells purified as described above were cultured at 0.5x10⁶ cells/ mL in SF Complete (αMEM with nucleosides, 50 µM B-mercaptoethanol, 1: 100 insulin, transferring, selenium stock (Gibco), 150 µg/ mL additional transferrin, 5 mg/ mL bovine serum albumin) with 4% autologous serum with or without the addition of 20 ng/ mL human IL-21 for 4 days at 37°C. On day 4 cells were harvested, counted on the hemocytometer and washed in Hank's Buffered Salt Solution (HBSS without Ca or Mg) with 5% fetal bovine serum (FBS), now called HBSSF. Cell pellets were re-suspended in HBSSF to .5x10⁶ cells/ mL.

Breast cancer target cell lines, including BT-474, or MCF-7, were labeled with 10 µM calcein in HBSSF for 1 hour at 37°C. Targets were then washed in 10 volumes of HBSSF at 1100 rpm for 8 minutes. Cell pellets were re-suspended in HBSSF to 50,000 cells/ mL. MNC effector cells pretreated with or without human IL-21 for 4 days as described above were centrifuged at 1100 rpm for 8 minutes and cell pellets re-suspended in HBSSF to about 0.5x10⁶ cells/ mL. Targets were serially diluted 1:3 in 96-well round bottom plates in duplicates. 100 µl targets were added to each well in the presence of either 2 µg/ mL human IgG, or 10, 5, 2.5, 1.25, 62, 31 µg/ mL herceptin. 96- well plates were centrifuged at 500 rpm for 3 minutes. Plates were incubated at 37°C for 3 hours and then centrifuged at 1000 rpm for 5 minutes. 100 µl supernatant from each well was transferred to 96 - well flat-bottomed plates and read on the Wallac fluorometer at 485/535 with 1 second intervals.

Using either BT-474 or MCF-7 cell lines as targets in an ADCC assay, MNCs pretreated with IL-21 have more activity at all concentrations of trastuzumab tested than untreated MNCs or when IgG is present in the assay. At an effector:target (E:T) of 3, the cytolytic activity is maximal at 5 µg/mL trastuzumab when BT-474s are used. At an E:T of 3, the cytolytic activity is maximal at 1.25 µg/mL when MCF-7s are used.

### D.

Peripheral blood was collected from cynomolgus monkeys into 5 ml tubes with lithium heparin and stored at room temperature until sample processing. Samples were diluted with PBS containing 1 mM EDTA, and the mononuclear cell (MNC) fraction was collected by centrifugation over 95% Ficoll. After washing, the cells were cultured for 3 days in growth media containing rIL-21 20 ng/ml or control media. After incubation, the cells were washed and counted, and aliquots were stained for immunophenotyping by flow cytometry. An aliquot of cells was used to perform antibody-dependent cellular cytotoxicity assays as follows. BT-474 breast cancer target cells were loaded with Calcein-AM dye for 60 minutes at 37 °C, washed, and 1000 target cells were placed into wells containing 2 µg/ml Herceptin and either 50,000, 25,000, 12,500, 6250, 3125, 1563, or 781 MNCs. The assays were incubated for 3 h. in the dark at 37 °C. Following this incubation, the release of Calcein-AM into supernatants was measured, and specific lysis was calculated based on release by total (detergent) lysis and the non-specific release in the absence of any MNC effector cells. The experiment was repeated twice for each of 8 cynomolgus monkey donors. Data were presented as percentage specific lysis per MNC at an E:T ratio of 25, or the data were normalized to reflect the actual NK cell numbers in the MNC preparation, based on the flow cytometry analysis. For NK-adjusted data, the E:T ratio was fit against percentage lysed using a 4-parameter sigmoidal curve, and the Hill equation was used to determine percentage lysed at an E:T ratio of 3 NKs per BT-474 target.

In vitro treatment of cynomolgus monkey MNCs with rIL-21 increased the activity in Herceptin-mediated ADCC assays using BT-474 breast cancer targets. Some animals had a larger response to rIL-21 than others, and this variable response was consistent by animal in repeated experiments. A mixed effects model was fit using Proc MIXED in SAS@ (Littell et al. SAS System for Mixed Models; SAS Institute, 1996) with treatment as a fixed effect and random effects for donor and treatment by donor interaction. The Kenward Roger option in SAS® was used to determine the denominator degrees of freedom for the calculation of the P-value for treatment. The treatment term was highly significant.

**Table 19 ADCC activity from cynolomolgus MNC preparations against BT-474 breast cancer targets. Endpoint is percentage lysed at an E:T ratio of 25.**

| | Controls-no treatment | rIL-21 pre-treatment | | |
|---|---|---|---|---|
| Animal | Cont-run1 | Cont-run2 | rIL21-run1 | rIL21-run2 |
| A | 15.10089 | | 34.57907 | |
| B | 4.576255 | 6.457869 | 17.69686 | 15.00928 |
| C | 12.60543 | 4.191629 | 29.77582 | 20.20712 |
| D | 25.48322 | 16.04117 | 32.51966 | 32.08234 |
| E | 20.77167 | 6.569754 | 32.82285 | 26.04751 |
| F | 15.85598 | 4.479947 | 22.39635 | 12.16969 |
| G | 31.07411 | 18.44403 | 54.5077 | 37.00462 |
| H | 26.21558 | 7.980985 | 29.85094 | 20.00046 |

**Table 20 ADCC activity from cynomolgus MNC preparations against BT-474 brast cancer targets. Endpoint is percentage lysed at an NK-adjusted E:T ratio of 3.**

| | Controls-no treatment rIL-21 | pre-treatment | | |
|---|---|---|---|---|
| Animal | Cont | Cont | IL-21 | IL-21 |
| A | 17.56976 | | 36.4764 | |
| B | 5.349258 | 7.43626 | 22.17732 | 17.10627 |
| C | 13.51977 | 3.109 | 31.55683 | 26.31246 |
| D | 24.98626 | 15.87297 | 31.02951 | 31.07489 |
| E | 19.4483 | 6.177042 | 38.98066 | 24.94877 |
| F | 16.14824 | 4.779288 | 24.31473 | 15.74664 |
| G | 31.54203 | 17.95528 | 53.47342 | 35.68798 |
| H | 23.98745 | 8.451089 | 32.60043 | 16.41115 |

### Example 7

### CD4/CD8 Depletion Mouse Model

Depletion of cells using antibodies against cell surface receptors has been used for many years to understand the specific roles for these cells in immune mechanisms. Antibodies against CD4 and CD8 antigens on T lymphocytes when injected into mice deplete specific T cell subsets by a mechanism involving ADCC and complement. Low dose antibodies are injected into mice to deplete between 20-50% of CD4 or CD8 T cells. Groups of mice are given IL-21 and its ability to enhance depletion is studied by following T cells by flow cytometry. Increased depletion of T cells with IL-21 indicates the ability of IL-21 to enhance antibody-mediated depletion of cells *in vivo*.

Rat anti-mouse CD4 (clone GK1.5, ATCC) or Rat anti-mouse CD8 (clone 53-6.72, ATCC) are used for depletion studies. Groups of 8-12 weeks old C57BL/6 mice (Charles River Laboratories) are injected i.p. with control antibody, 5-50 µg of anti-CD4 or anti-CD8 mAb on day 0. Groups of mice receive either PBS or 25 µg mIL-21 starting at two days prior to bleeds until day one i.p. Mice are bled on days 1, 4 and 7. Blood CD4 and CD8 T cells numbers are assayed by flow cytometry.

Increased depletion of T cells with IL-21 indicates ability of IL-21 to enhance antibody-mediated depletion of cells *in vivo*, suggesting that IL-21 can enhance antibody mediated effects.

### Example 8

### Lung clearance assay

Lung clearance assays have been used to study function of NK cells *in vivo*. Chromium-51 (⁵¹Cr) labelled RAJI cells are inject i.v. into mice. Groups of mice receive PBS, mIL-21, rituximab alone or rituximab + IL-21. Mice are sacrificed 5-8 hours after i.v. injection and lungs assayed for the amount of radioactivity using a gamma-counter. Decrease in radioactivity in the lung is an indicator of increased clearance (killing) of tumor cells by NK cells. Ability of IL-21 to enhance clearance of tumor cells in the presence of rituximab is indicative of IL-21's ability to enhance antibody mediated lytic activity *in vivo.*

RAJI cells are labeled with 100 µCi ⁵¹Cr for 2 hours at 37°C. Cells are washed twice with PBS and resuspended in sterile PBS, pH 7.2. Mice are injected i.v. with 10 million labeled RAJI cells at time 0 (t=0). Groups of mice receive 20 µg control antibody or rituximab i.p. at t=10min. Groups of mice receive PBS or 25 µg mIL-21 at t=-24hrs, t=0hrs and t=4hrs. Mice are sacrificed between 5-8 hours after tumor injection, lungs isolated and counted on a gamma-counter. Radioactivity is plotted as percentage of control injections (labeled cells alone).

Decreased radioactivity in the lung is an indicator of increased clearance (killing) of tumor cells by NK cells. The ability of IL-21 to enhance clearance of tumor cells in the presence of rituximab is indicative of its ability to enhance antibody-mediated lytic activity *in vivo.*

### Example 9

### Raji/SCID Macrophage Depletion Study

The combination of IL-21+rituximab (rituximab) has a synergistic antitumor activity in a disseminated Raji/SCID tumor model. ADCC is thought to play an important role in the antitumor activity of rituximab *in vivo,* and macrophages are important effector cells in this process. IL-21 may influence the ADCC activity of macrophages in mice, leading to the synergy with rituximab. In order to test the importance of macrophages in the antitumor effect of IL21+ rituximab, macrophages will be depleted in mice, using clodronate liposomes (Sigma, St. Louis, MO). The experiment demonstrates that macrophages are critical for the synergistic antitumor activity of IL21+ rituximab by demonstrating that mice depleted of this cell population have hortened survival relative to non-depleted mice.

To study the importance of macrophages in the antitumor activity of IL21+ rituximab against Raji cells in SCID mice, the following experiment was performed. Treatment with rituximab was delayed to reduce its efficacy (Funakoshi, Longo et al. Blood, 83(10):2787-94, 1994), and injected mIL-21 for 5 consecutive days bracketing the first rituximab injection. HS-Sultan and Raji cells were used because they do not signal via STAT1 or STAT3 and they are not growth inhibited by IL-21 *in vitro* or *in vivo*).

| Group | Strain | #Mice | Treatment |
|---|---|---|---|
| 1.) | SCID | 10 (1481-90) | Clodronate Liposomes+IL-21+rituximab |
| 2.) | SCID | 10 (1491-1500) | PBS Liposomes+IL-21+rituximab |
| 3.) | SCID | 9 (1501-09) | Clodronate Liposomes+ rituximab |
| 4.) | SCID | 9 (1510-18) | PBS Liposomes+ rituximab |
| 5.) | SCID | 9 (1519-28) | Clodronate Liposomes+PBS |

1 x 10⁶ Raji cells injected IV on day 0 of study
100 µg IL-21 given by IP route on days 3-7
20 µg rituximab given by IP route on day 5, day 9, day 13, day 17, and day 21. Liposomes given IV:
day 3 - 0.2 ml 100 % liposomes
day 9 - 0.2 ml 50% liposomes
day 15 - 0.2 ml 50% liposomes
day 21 - 0.2 ml 50% liposomes

Figure 1 shows that macrophage depletion with clodronate liposomes (e.g.Clod.IL21+R) dramatically reduced the survival benefit for SCID mice bearing Raji lymphoma cells when compared to mice injected with PBS liposomes. Macrophage depleted mice treated with IL-21+rituximab (Clod.IL21+R) or rituximab (Clod.R) had significantly shorter survival (mean time to death) compared to non-depleted mice (PBS.IL21+R and PBS.R respectively).

### Example 10

### Tumor clearance after IL-21 + rituximab in SCID mice with depleted granulocytes

IL-21 along with rituximab is able to efficiently clear RAJI tumor cells *in vivo* better then rituximab alone. RAJI cells will be injected into CB17 SCID mice which have depleted granulocytes. The effect of rituximab alone or in combination with IL-21 was studied.

CB17-scid mice were injected with 1 x 10⁶ of Raji cells IV. In addition, some of the mice were injected with monoclonal antibody Gr-1 (BD Biosciences, Palo Alto, CA). Mice will be treated with 20µg of Rituxan, 100µg mIL-21 or a combination of Rituxan and mIL-21 via IP injections.

Mice were monitored for 1) consistent or rapid body weight loss of 20%, 2) paralysis or inability to maintain an upright position or move, 3) labored breathing - especially if accompanied by nasal discharge or cyanosis, 4) lethargic or failure to respond to gentle stimulis. Mice meeting the above criteria were euthanized.

Ab treatments were dosed for groups 1-6 on days 5, 9, 13, 17 & 21. Groups 7-10 were treated on days 12, 19, 26, 33 and 40. Protein was dosed on days 3-7 for groups 1-6 and on days 10-14 for groups 7-10.

| Mice | # | Depletion | Ab treatment | Protein |
|---|---|---|---|---|
| 1) C.b-17 SCID | 8 | none | PBS | PBS |
| 2) C.B-17 SCID | 8 | none | 20µg rituximab | PBS |
| 3)C.B-17 SCID | 8 | none | 20µg rituximab | 100 ug IL-21 |
| 4) C.B-17 SCID | 8 | Gr-1 | PBS | PBS |
| 5) C.B-17 SCID | 8 | Gr-1 | 20µg rituximab | PBS |
| 6) C.B-17 SCID | 8 | Gr-1 | 20µg rituximab | 100 ug IL-21 |

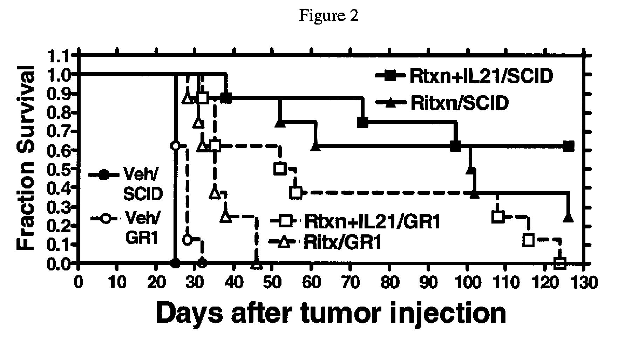

Figure 2 shows that the synergistic antitumor activity of IL-21+rituximab is compromised by granulocyte- depletion with anti-Gr-1 MAb. The survival of Raji bearing SCID mice (Fraction Surviving at 100 days) is significantly reduced for granulocyte-depleted SCID mice (dashed lines) when compared to non-depleted mice (solid lines).

### Example 11

### IL-21 in combination with anti-CTLA4 antibodies

### A. RENCA cell tumor model

To test whether IL-21 in combination with anti-CTLA4 mAb has effects on tumor growth in mice, a RENCA cell tumor model was used. Renal cell carcinoma mouse models using Renca cell injections have been shown to establish renal cell metastatic tumors that are responsive to treatment with immunotherapeutics such as IL-12 and IL-2 (Wigginton et al., J. of Nat. Cancer Inst. 88:38-43, 1996). Groups of mice were injected s.c with the RENCA tumor on Day 0. Mice were then injected with vehicle alone, 50ug or 100ug anti-CTLA4 MAb (clone 9H10, eBiosciences, San Diego, CA), 25ug mIL-21 alone or 50ug or 100ug anti-CTLA4 in combination with 25ug mIL-21. A low dose of 25ug mIL-21 that normally does not have potent antitumor effect in this model was used.

Ten-week old female BALB/c mice (Charles River Laboratories) were injected SC on the right flank with 0.1 x 10⁶ RENCA cells on Day 0. Groups of mice received vehicle alone (PBS, pH 7.2) or 25ug mIL-21 on Days 5-9, 19-23. Separate groups received either 50ug or 100ug anti-CTLA-4 MAb alone on Days 0, 4 and 8 or received anti-CTLA4 MAb (50ug or 100ug) on Days 0, 4 and 8 in combination with 25ug mIL-21 on Days 5-9, 19-23. All injections were administered intraperitoneally. Tumor growth was monitored 3X/week for 5 weeks using caliper measurements. Tumor volume was calculated using the formula ½*(B)²*L (mm³).

Injection of mIL-21 alone or the two concentrations of anti-CTLA4 MAb alone had no substantial effect on tumor growth. In contrast combination of mIL21 with anti-CTLA4 MAb at either concentration showed significant decrease in tumor volume compared to controls (Figure 1). These data suggest that the combination of IL21 with anti-CTLA4 MAb has synergistic antitumor activity and is a possible combination therapeutic for cancer.

### B. Therapeutic administration of mouse IL-21 in combination with anti-mouse CTLA4 inhibits tumor growth in the RENCA model

### To test if combining IL-21 with anti-CTLA4 mAb has effects on tumor growth in mice when administered using a therapeutic regimen, groups of mice are injected s.c with the RENCA tumor on Day 0. Mice are then injected with vehicle alone, 50ug or 100ug anti-CTLA4 mAb (clone 9H10, eBiosciences), 25ug mIL-21 alone or 50ug or 100ug anti-CTLA4 MAb in combination with 25ug mIL-21 starting at a tumor volume of 60-80mm³. A low dose of 25ug mIL-21 that normally does not have potent antitumor effect in this model is used. Anti-CTLA4 mAb is administered on Days 1, 5 , 9 and 13 after tumor volume of 60-80mm³ has been reached. mIL-21 is injected on Days 5-9, 19-23 or from Days 1-10 after tumor volume has reached 60-80mm³. Antitumor effects seen in the groups combining mIL-21 and anti-CTLA4 MAb suggest a synergistic antitumor effect in this model when administered in a therapeutic regimen.

Ten-week old female BALB/c mice (Charles River Laboratories) are injected SC on the right flank with 0.1 x 10⁶ RENCA cells on Day 0. Groups of mice receive vehicle alone (PBS, pH 7.2) or 25ug mIL-21 on Days 5-9, 19-23 or on days 1-10 after tumor volume has reached 60-80mm³. Separate groups receive either 50ug or 100ug anti-CTLA MAb alone on Days 1, 5, 9 and 13 or receive anti-CTLA4 MAb (50ug or 100ug) on days 1, 5, 9 and 13 in combination with 25ug mIL-21 on days 5-9, 19-23 or days 1-10 after tumor volume has reached 60-80mm³. All injections are administered intraperitoneally. Tumor growth is monitored 3X/week for 5 weeks using caliper measurements. Tumor volume is calculated using the formula ½*(B)²*L (mm³).

Antitumor effects seen in the groups combining mIL-21 and anti-CTLA4 MAb suggest a synergistic antitumor effect in this model when administered in a therapeutic regimen. These data suggest that the combination of IL21 with anti-CTLA4 mAb has synergistic antitumor activity and is a possible combination therapeutic for cancer.

### C. Combination treatment with mIL-21 and anti-mouse CTLA4 inhibits tumor growth in the E.G7 thymoma model

To test if combination of mIL-21 and anti-CTLA4 MAb induces antitumor activity, groups of mice are injected s.c with the E.G7 tumor on Day 0 (Shrikant, P and Mescher, M,. J. Immunology 162:2858-2866, 1999). Mice are then injected with vehicle alone, 50ug or 100ug anti-CTLA4 mAb (clone 9H10, eBiosciences), 25ug mIL21 alone or 50ug or 100ug anti-CTLA4 in combination with 25ug mIL21. A low dose of 25ug mIL21 that normally does not have potent antitumor effect in this model is used. Anti-CTLA4 mAb is administered on Days 0, 4 and 8. mIL21 is injected on Days 5-9, 19-23 or on Days 2-20 every other day (EOD). Antitumor effects seen in the groups combining mIL21 and CTLA4 suggest a synergistic antitumor effect in this model.

Ten-week old female C57BL/6 mice (Charles River Laboratories) are injected SC on the right flank with 0.4 x 10⁶ E.G7 cells (ATCC No. CRL-2113) on Day 0. Mice are then injected with vehicle alone, 50ug or 100ug anti-CTLA4 mAb (clone 9H10, eBiosciences), 25ug mIL-21 alone or 50ug or 100ug anti-CTLA4 MAb in combination with 25ug mIL-21. A low dose of 25ug mIL-21 that normally does not have potent antitumor effect in this model is used. Anti-CTLA4 mAb is administered on Days 0, 4 and 8. mIL-21 is injected on Days 5-9, 19-23 or on Days 2-20 every other day (EOD). Intra-peritoneal injections were given in a total volume of 200ul. All reagents are given by intraperitoneal injections. Tumor growth is monitored 3X/week for 4 weeks using caliper measurements. Tumor volume was calculated using the formula ½*(B)²*L (mm³).

Antitumor effects seen in the groups combining mIL-21 and anti-CTLA4 MAb suggest a synergistic antitumor effect in this model. These data suggest that the combination of IL-21 with anti-CTLA4 mAb has synergistic antitumor activity and is a possible combination therapeutic for cancer.

### D. Combination treatment with mIL-21 and anti-mouse CTLA4 MAb inhibits tumor growth in the B16 melanoma model

To test if combination of mIL-21 and anti-CTLA4 MAb induces antitumor activity in other tumors, groups of mice are injected s.c with the B16-F10 melanoma cells (ATCC No. CRL-6475)on Day 0. Mice are then injected with vehicle alone, 50ug or 100ug anti-CTLA4 mAb (clone 9H10, eBiosciences), 25ug mIL-21 alone or 50ug or 100ug anti-CTLA4 MAb in combination with 25ug mIL-21. Anti-CTLA4 mAb is administered on Days 0, 4 and 8. mIL-21 is injected on Days 5-9, 19-23 or on Days 2-20 every other day (EOD). Antitumor effects seen in the groups combining mIL-21 and anti-CTLA4 MAb suggest a synergistic antitumor effect in this model.

Ten-week old female C57BL/6 mice (Charles River Laboratories) are injected SC on the right flank with 0.5 x 10⁶ B16 melanoma cells on Day 0. Mice are then injected with vehicle alone, 50ug or 100ug anti-CTLA4 mAb (clone 9H10, eBiosciences), 25ug mIL-21 alone or 50ug or 100ug anti-CTLA4 MAb in combination with 25ug mIL-21. Anti-CTLA4 mAb is administered on Days 0, 4 and 8. mIL21 is injected on Days 5-9, 19-23 or on Days 2-20 every other day (EOD). Intra-peritoneal injections were given in a total volume of 200ul. All reagents are given by intraperitoneal injections. Tumor growth is monitored 3X/week for 4 weeks using caliper measurements. Tumor volume was calculated using the formula ½*(B)²*L (mm³).

Antitumor effects seen in the groups combining mIL-21 and anti-CTLA4 MAb suggest a synergistic antitumor effect in this model. These data suggest that the combination of IL-21 with anti-CTLA4 mAb has synergistic antitumor activity and is a possible combination therapeutic for cancer.

From the foregoing, it will be appreciated that, although specific embodiments of the invention have been described herein for purposes of illustration, various modifications may be made without deviating from the spirit and scope of the invention. Accordingly, the invention is not limited except as by the appended claims.
1. A method of treating cancer in a subject comprising co-administering a therapeutically effective amount of a monoclonal antibody and a therapeutically effective amount of an IL-21 polypeptide or fragment of an IL-21 polypeptide as shown in SEQ ID NO:2 from amino acid residue 30 to residue 162.
2. A method treating cancer in a subject comprising co-administering a therapeutically effective amount of an anti-CD20 monoclonal antibody and a therapeutically effective amount of an IL-21 polypeptide or a fragment of an IL-21 polypeptide as shown in SEQ ID NO:2 from amino acid residue 30 to residue 162.
3. The method of clause 2, wherein the monoclonal antibody is rituximab.
4. The method of clause 2, wherein the cancer is non-Hodgkin's lymphoma.
5. The method of clause 2, wherein the subject is a human patient.
6. The method of clause 3, wherein the rituximab and IL-21 polypeptide are administered once weekly for up to eight consecutive weeks.
7. The method of clause 3, wherein the rituximab is administered once weekly and the IL-21 polypeptide is administered up to five times weekly for up to eight consecutive weeks.
8. The method of clause 3, wherein the IL-21 polypeptide dose is from 10 to 500 µg/kg/dose.
9. The method of clause 5, wherein the patient has previously been treated with rituximab and showed no appreciable tumor remission or regression.
10. The method of clause 5, wherein the patient has relapsed after receiving rituximab therapy.
11. A method treating cancer in a subject comprising co-administering a therapeutically effective amount of an anti-CD20 monoclonal antibody and a therapeutically effective amount of an IL-21 polypeptide or a fragment of an IL-21 polypeptide as shown in SEQ ID NO:2 from amino acid residue 30 to residue 162, wherein administering the IL-21 results in an optimal immunological response.
12. A method treating cancer in a subject comprising co-administering a monoclonal antibody that binds to a Her-2/neu receptor and an IL-21 polypeptide or a fragment of an IL-21 polypeptide as shown in SEQ ID NO:2 from amino acid residue 30 to residue 162.
13. The method of clause 12, wherein the subject is a human patient.
14. The method of clause 13, wherein the monoclonal antibody is trastuzumab.
15. A method of treating cancer in a subject comprising co-administering a monoclonal antibody that binds to a cytotoxic T lymphocyte-associated antigen 4 (CTLA-4) and an IL-21 polypeptide or a fragment of an IL-21 polypeptide as shown in SEQ ID NO:2 from amino acid residue 30 to residue 162 or.
16. The method of clause 15, wherein the subject is a human patient.
17. The method of clause 16, wherein the anti-CTLA-4 monoclonal antibody is administered at a dose of 3 mg/kg every three weeks for four cycles and the IL-21 polypeptide or fragment is administered one to five times weekly for up to eight weeks.
18. The method of clause 17, where in the IL-21 polypeptide dose is from 10 to 500 µg/kg/dose.

## Claims

1. A monoclonal antibody that binds to binds to cytotoxic T lymphocyte-associated antigen 4 (CTLA-4), programmed death-1 (PD-1), B and T lymphocyte attenuator (BTLA), IL-10, TGFβ, or CD25, and an IL-21 polypeptide or fragment of an IL-21 polypeptide as shown in SEQ ID NO:2 from amino acid residue 30 to residue 162, for use in treating cancer in a subject.

2. The monoclonal antibody and IL-21 polypeptide or fragment for use according to Claim 1, wherein the monoclonal antibody binds to a cytotoxic T lymphocyte-associated antigen 4 (CTLA-4).

3. The monoclonal antibody and IL-21 polypeptide or fragment for use according to Claim 2, wherein the subject is a human patient.

4. The monoclonal antibody and IL-21 polypeptide or fragment for use according to Claim 2 or 3, wherein the anti-CTLA-4 monoclonal antibody is administered at a dose of 3 mg/kg every three weeks for four cycles and the IL-21 polypeptide or fragment is administered one to five times weekly for up to eight weeks.

5. The monoclonal antibody and IL-21 polypeptide or fragment for use according to Claim 2, wherein the cancer is selected from the group consisting of melanoma, prostate cancer, ovarian cancer, renal cell cancer, and thymoma.

6. The monoclonal antibody and IL-21 polypeptide or fragment for use according to Claim 5, wherein the cancer is melanoma.

7. The monoclonal antibody and IL-21 polypeptide or fragment for use according to any previous claim, wherein the IL-21 polypeptide dose is from 10 to 500µg/kg/dose.

8. The monoclonal antibody and IL-21 polypeptide or fragment for use according to any previous claim, wherein the patient has previously not responded to monoclonal antibody therapy alone or in combination with other treatment regimes.
